# EUROPEAN PATENT SPECIFICATION

(11) **EP 1 373 571 B1**
(45) Date of publication and mention of the grant of the patent: **22.11.2006**
(21) Application number: 02718168.4
(22) Date of filing: 01.03.2002
(51) Int. Cl.: C12Q 1/68

(54) **FUNCTIONAL SURFACE DISPLAY OF POLYPEPTIDES**
FUNKTIONELLES OBERFLÄCHENDISPLAY VON POLYPEPTIDEN
AFFICHAGE EN SURFACE FONCTIONNEL DE POLYPEPTIDES

(30) Priority: 02.03.2001 EP 01105129
(43) Date of publication of application: 02.01.2004
(73) Proprietor: Universität des Saarlandes, Wissens- und Technologietransfer GmbH, 66123 Saarbrücken (DE)
(72) Inventor: JOSE, Joachim, 66123 Saarbrücken (DE); HANNEMANN, Franck, 66125 Saarbrücken (DE); BERNHARDT, Rita, 66129 Saarbrücken (DE)
(74) Representative: Weiss, Wolfgang
(86) International application number: PCT/EP2002/002246
(87) International publication number: WO 2002/070645

(56) References cited:
- LATTEMANN CLAUS T ET AL: "Autodisplay: Functional display of active beta-lactamase on the surface of Escherichia coli by the AIDA-I autotransporter." JOURNAL OF BACTERIOLOGY, vol. 182, no. 13, July 2000 (2000-07), pages 3726-3733, XP002231687 ISSN: 0021-9193
- JUNG H. ET AL.: "surface display of Zymonas mobilis levansucrase by using the ice-nucleation protein of pseudomonas sysringae" NATURE BIOTECHNOLOGY, vol. 16, 1998, XP001145493
- KONIECZNY MARC P J ET AL: "Cell surface presentation of recombinant (poly-) peptides including functional T-cell epitopes by the AIDA autotransporter system." FEMS IMMUNOLOGY AND MEDICAL MICROBIOLOGY, vol. 27, no. 4, April 2000 (2000-04), pages 321-332, XP002231688 ISSN: 0928-8244
- STAHL S ET AL: "Bacterial surface display: trends and progress" TRENDS IN BIOTECHNOLOGY, ELSEVIER PUBLICATIONS, CAMBRIDGE, GB, vol. 15, no. 5, 1 May 1997 (1997-05-01), pages 185-192, XP004059848 ISSN: 0167-7799
- JOSE J. ET AL: "functional display of active bovine adrenoxin on the surface of E. Coli by chemical incorporation of the 2Fe-2S cluster " CHEMBIOCHEM, vol. 2, 2001, pages 695-701, XP002231689

## Description

The present invention relates to a method for the display of recombinant functional polypeptides containing a prosthetic group and/or a plurality of subunits on the surface of a host cell using the transporter domain of an autotransporter.

Surface display of active proteins on living cells provides several advantages in biotechnological applications. Using such cells as whole cell biocatalysts or whole cell biofactories, a substrate to be processed does not need to cross a membrane barrier but has free access. Moreover, being connected to a carrier (the cell) or let us call it a biological matrix, the surface-displayed biocatalyst can be purified, stabilized and applied to industrial processes more convenient than it is in most cases as a free molecule. Using cellular surface display in creating and screening peptide or protein libraries in order to perform laboratory evolution has another benefit. By selecting the correct structure expressed at the surface, the corresponding intrinsic label (gene) is co-selected and can be used in further studies and applications. Therefore the need of systems that allow the surface display of a most broad spectrum of different proteins is obvious and gains more and more importance in typical biochemical or bioorganical application fields as e.g. enzyme engineering or drug discovery.

Therefore, surface display of recombinant proteins on living cells bears promising options towards future biotechnology applications e.g. whole cell biofactories or taylor-made enzymes by laboratory evolution (1). Different systems have been applied for the surface display of heterologous proteins in yeast (2,3), gram-positive (4,5), and gram-negative bacteria (6). Beside other systems (7-14), autodisplay is a very elegant way to express a recombinant protein on the surface of a gram-negative bacterium (15,16). Autodisplay is based on the secretion mechanism of the autotransporter family of proteins (17-19). These proteins are synthesized as polyprotein precursors that contain structural requirements sufficient for secretion (20). They cross the inner membrane using a typical signal peptide at the very N-terminus. Arrived in the periplasm, the C-terminal part of the precursor folds into the outer membrane as a porin-like structure, a so-called β-barrel (15,21,22). Through this pore, the N-terminal attached passenger domain is translocated to the surface. There, it might be cleaved off- either autoproteolytically or by an additional protease- or remains anchored to the cell envelope by the transporter domain (23). Replacing the natural passenger by a recombinant protein results in its proper surface translocation (15,16,24-27). For this purpose an artificial precursor must be constructed by genetic engineering, consisting of a signal peptide, the recombinant passenger, the β-barrel and a linking region in between, which is needed to achieve full surface access (21). The AIDA-I autotransporter was successfully used in this way for efficient surface display of various passenger domains (15,16,27,28). Up to now, however, the autotransporter mediated surface display has been restricted to monomeric proteins that were devoid of any non-proteinaceous cofactors. This can be due to the fact that a polypeptide chain to be transported by the autotransporter pathway must be in an relaxed, unfolded conformation.

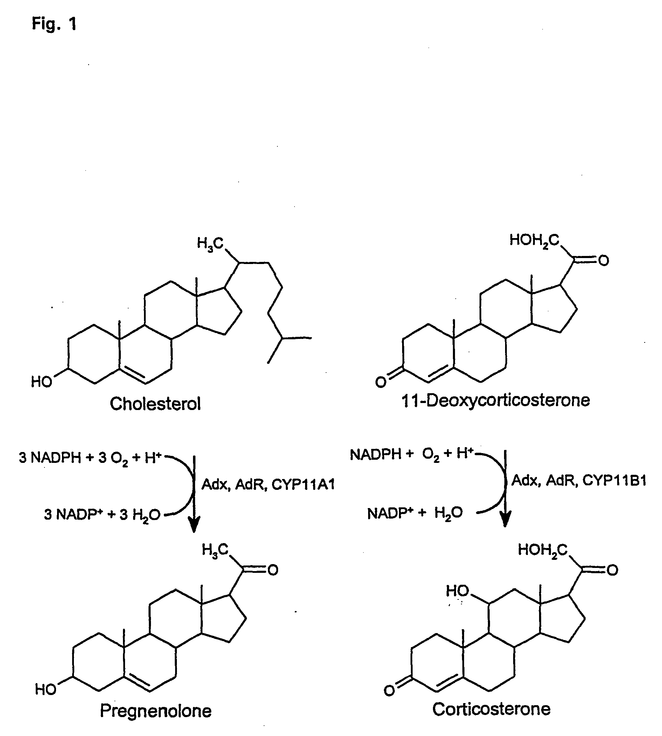

The ferredoxin from bovine adrenal cortex, termed adrenodoxin (Adx), belongs to the [2Fe-2S] ferredoxins, a family of small acidic iron-sulfur proteins, which can be found in bacteria, plants, and animals (29). It plays an essential role in electron transport from adrenodoxin reductase (AdR) to mitochondrial cytochromes P450, which are involved in the synthesis of steroid hormones (Fig. 1) (30). Moreover, cytochromes P450 play an essential role in the biosynthesis of prostaglandins or secondary metabolites of plants and microorganisms, as well as in the detoxification of a wide range of foreign compounds as drugs or chemical pollutants (41). The iron-sulfur cluster of Adx is coordinated by four sulfur atoms from side chains of four of its five cysteine residues (31). Bovine adrenodoxin is encoded by a nuclear gene, synthesized in the cytoplasm and processed upon mitochondrial uptake. The mechanism of iron-sulfur cluster incorporation is still not clear, however, during heterologous expression in *E. coli*, it can be assembled in the cytoplasm as well as in the periplasm (32).

In the present application, we report on the construction of a fusion protein, comprised of a signal peptide, a monomeric Adx and the transporter domain of AIDA-I and its efficient and stable surface display. At the surface the iron-sulfur cluster could be incorporated by a one step procedure, yielding functional Adx. We show for the first time that autodisplay can be applied to proteins that contain inorganic cofactors and that these proteins are freely accessible at the cell surface, even for large ligands or partner proteins as adrenodoxin reductase or cytochromes P450. Hence, we could obtain e.g. a whole cell biocatalyst system comprising Adx, adrenodoxin reductase and P450 CYP11A1 that effectively synthesized pregnenolone from cholesterol. Addition of artificial membrane constituents or detergents, which was indispensable before to obtain functional steroidal P450 enzymes, was not necessary. This investigation opens the door for further dimensions in the application of autodisplay, either in the evolutive design of catalytic biomolecules or for new whole cell factories.

Thus, a first aspect of the present invention is a method for displaying a recombinant polypeptide containing a prosthetic group on the surface of a host cell comprising the steps:
(a) providing a host cell transformed with a nucleic acid fusion operatively linked with an expression control sequence; said nucleic acid fusion comprising:
   (i) a portion encoding a signal peptide,
   (ii) a portion encoding the recombinant polypeptide to be displayed,
   (iii) optionally a portion encoding a protease recognition site,
   (iv) a portion encoding a transmembrane linker, and
   (v) a portion encoding the transporter domain of an autotransporter,
(b) culturing the host cell under conditions wherein the nucleic acid fusion is expressed and the expression product comprising the recombinant polypeptide is displayed on the surface of the host cell, and
(c) contacting the recombinant polypeptide with a prosthetic group under conditions wherein the prosthetic group combines with the recombinant polypeptide and a functional recombinant polypeptide containing the prosthetic group is formed.

According to the present invention, it is possible to display polypeptides, e.g. enzymes requiring for functionality a prosthetic group on the surface of a host cell. The prosthetic group may be combined with the recombinant polypeptide on the surface of the host cell, on a membrane preparation derived from the host cell or after cleavage of the recombinant polypeptide from the host cell or a membrane preparation thereof. The procedure wherein the prosthetic group is combined with the recombinant polypeptide may comprise thermal treatment, e.g. heating and/or chemical treatment, e.g. reduction, oxidation and/or pH adjustment. It should be noted that also a plurality of prosthetic groups which may be identical or different may be combined with the recombinant polypeptide.

The prosthetic group may be selected from any non-proteinaceous component which is known to function as a prosthetic group. For example, the prosthetic group may comprise an inorganic component such as a metal which may be present as a metal ion. For example the metal may be selected from heavy metals such as cobalt, nickel, manganese, copper and iron. Preferred examples of prosthetic groups are [2Fe-2S] clusters, [4Fe-4S] clusters and metal porphyrin, e.g. heme groups.

Furthermore, a prosthetic group may be selected which comprises an organic component, e.g. a coenzyme. Examples of such prosthetic groups are flavin containing groups, e.g. FMN or FAD, nicotin containing groups, e.g. NAD, NADH, NADP or NADPH, biotin, α2-microglobulin, thiamine pyrophosphate, coenzyme A, pyridoxal phosphate, coenzyme B12, biocytine, tetrahydrofolate and lipoic acid.

A further embodiment of the present invention relates to a method for displaying a recombinant multimeric polypeptide on the surface of a host cell comprising the steps:
(a) providing a host cell transformed with a nucleic acid fusion operatively linked with an expression control sequence said nucleic acid fusion comprising:
   (i) a portion encoding a signal peptide,
   (ii) a portion encoding at least one subunit of the multimeric polypeptide to be displayed,
   (iii) optionally a portion encoding a protease recognition site,
   (iv) a portion encoding a transmembrane linker, and
   (v) a portion encoding the transporter domain of an autotransporter,
(b) culturing the host cell under conditions wherein the nucleic acid fusion is expressed and the expression product comprising the subunit of the multimeric recombinant polypeptide is displayed on the surface of the host cell, and
(c) combining the displayed subunit with at least one further subunit of the multimeric recombinant polypeptide and forming a functional multimeric polypeptide on the surface of the host cell.

The multimeric recombinant polypeptide may be a homodimer, i.e. a polypeptide consisting of two identical subunits or a homomultimer, i.e. a polypeptide consisting of three or more identical subunits. On the other hand, the multimeric recombinant polypeptide may be a heterodimer, i.e. a polypeptide consisting of two different subunits or a heteromultimer consisting of three or more subunits wherein at least two of these subunits are different. For example, the multimeric polypeptide is comprised of a plurality of subunits which form a "single" multimeric polypeptide or a complex of a plurality of functionally associated polypeptides which may in turn be monomeric and/or multimeric polypeptides. It should be noted that at least one subunit of the multimeric recombinant protein may contain at least one prosthetic group. Further, is should be noted that the nucleic acid fusion may encode a plurality of polypeptide subunits as a polypeptide fusion which when presented on the cell surface forms a functional multimeric polypeptide.

Homodimers or homomultimers may be formed by a spontaneous association of several identical polypeptide subunits displayed on the host cell membrane. Heterodimers or heteromultimers may be formed by a spontaneous association of several different polypeptide subunits displayed on the host cell membrane.

On the other hand, a multimeric recombinant polypeptide may be formed by an association of at least one polypeptide subunit displayed on the host cell membrane and at least one soluble polypeptide subunit added to the host cell membrane. The added subunit may be identical to the displayed subunit or be different therefrom.

The host cell used in the method of the present invention is preferably a bacterium, more preferably a gram-negative bacterium, particularly an enterobacterium such as *E. coli.*

According to the present invention, a host cell, particularly a host bacterium is provided which is transformed with at least one nucleic acid fusion operatively linked with an expression control sequence, i.e. a promoter and optionally further sequences required for gene expression in the respective host cell. Preferably, the nucleic acid fusion is located on a recombinant vector, e.g. a plasmid vector. In case a host cell transformed with several nucleic acid fusions is used, these nucleic acid fusions may be located on a single vector or on a plurality of compatible vectors. The nucleic acid fusion comprises (i) a portion encoding a signal peptide, preferably a portion coding for a gram-negative signal peptide allowing for transport into the periplasm through the inner cell membrane. Further, the nucleic acid fusion comprises (ii) a portion encoding the recombinant polypeptide to be displayed and (iii) optionally a portion encoding a protease recognition site, which may be a recognition site for an intrinsic protease, i.e. a protease naturally occurring in the host cell, or an externally added protease. For example, the externally added protease may be an IgA protease (cf. EP-A-0 254 090), thrombin or factor X. The intrinsic protease may be e.g. selected from OmpT, OmpK or protease X. Furthermore, the nucleic acid fusion comprises (iv) a portion encoding a transmembrane linker which is required for the presentation of the passenger polypeptide (ii) on the outer surface of the outer membrane of the host cell. Further, the nucleic acid fusion comprises (v) a transporter domain of an autotransporter.

Preferably a transmembrane linker domain is used which is homologous with regard to the autotransporter, i.e. the transmembrane linker domain is encoded by a nucleic acid portion directly 5' to the autotransporter domain. The length of the transmembrane linker is preferably 30-160 amino acids.

The length of the recombinant polypeptide to be displayed, i.e. the passenger polypeptide is preferably in the range of from 5-3000 amino acids, more preferably in the range from 10-1500 amino acids.

The transporter domain of the autotransporter according to the invention can be any transporter domain of an autotransporter and is preferably capable of forming a β-barrel structure. A detailed description of the β-barrel structure and preferred examples of β-barrel autotransporters are disclosed in WO97/35022 incorporated herein by reference. For example, the transporter domain of the autotransporter may be selected from the *E. coli* AIDA-I protein, the *Shigella flexneri* Sep A protein, the *Shigella flexneri* IcsA protein, the *E. coli* Tsh protein, the *Serratia marcescens* Ssp protein, the *Helicobacter mustelae* Hsr protein, the *Bordetella ssp.* Prn protein, the *Haemophilus influenzae* Hap protein, the *Bordetalla pertussis* Brk A protein, the *Helicobacter pylori* Vac A protein, the surface protein SpaP, rOmpB or SlpT from *Rickettsia,* the IgA protease from *Neisseria* or *Haemophilus* and variants thereof. More preferably the transporter domain of the autotransporter is the *E. coli* AIDA-I protein or a variant thereof, such as e.g. described by Niewert U., Frey A., Voss T., Le Bouguen C., Baljer G., Franke S., Schmidt MA. The AIDA Autotransporter System is Associated with F18 and Stx2e in Escherichia coli Isolates from Pigs Diagnosed with Edema Disease and Postweaning Diarrhea. Clin. Diagn. Lab. Immunol. 2001 Jan, 8(1):143-149;9.

Variants of the above indicated autotransporter sequences can e.g. be obtained by altering the amino acid sequence in the loop structures of the β-barrel not participating in the transmembrane portions. Optionally, the nucleic acid portions coding for the surface loops can be deleted completely. Also within the amphipathic β-sheet conserved amino exchanges, i.e. the exchange of an hydrophilic by another hydrophilic amino acid or/and the exchange of a hydrophobic by another hydrophobic amino acid may take place. Preferably, a variant has a sequence identity of at least 50% and particularly at least 70% on the amino acid level to the respective native sequence of the autotransporter domain at least in the range of the β-sheets.

A further aspect of the present invention relates to a host cell displaying a functional recombinant polypeptide on the surface thereof wherein the recombinant polypeptide contains a prosthetic group and wherein the recombinant polypeptide is preferably displayed by the transporter domain of an autotransporter. For example, the polypeptide may be selected from ferredoxins, e.g. adrenodoxin, P450 reductases, cytochrome b5, P450 enzymes, flavoproteins, e.g. oxidoreductases, dehydrogenases or oxidases, especially sugar oxidases, such as pyranose oxidase (Fig. 15), and any combinations thereof.

Still a further embodiment of the present invention is a host cell displaying a functional recombinant polypeptide on the surface thereof wherein the recombinant polypeptide is a multimeric polypeptide containing at least two polypeptide subunits and wherein at least one subunit of the multimeric polypeptide is preferably displayed by the transporter domain of an autotransporter.

Further, the invention relates to a membrane preparation which is derived from a host cell as described above wherein this membrane preparation displays a functional recombinant polypeptide containing a prosthetic group and/or being a multimeric polypeptide.

The method according to the invention and the host cells according to the invention can be used for a variety of different applications, e.g. as whole cell biofactories or membrane preparation biofactories for chemical synthesis procedures, e.g. for the synthesis of organic substances selected from enzyme substrates, drugs, hormones, starting materials and intermediates for syntheses procedures and chiral substances (cf. Roberts, Chemistry and Biology 6 (1999), R269-R272).

Furthermore, the cell or the membrane preparation of the invention may be used for a directed evolution procedure, e.g. for the development of new biocatalysts for the application in organic syntheses.

This is achieved in a particular embodiment by varying the amino acid sequence of a P450 enzyme or a flavoprotein via site-specific or random mutagenesis and by testing variant carrying cells or membrane preparations or libraries containing variant carrying cells or membrane preparations thereof using a certain chemical reaction with the help of suitable screening methods, in particular high throughput screening (HTS) methods for the conversion of a certain substrate.

In a further preferred embodiment libraries of variants produced by site-specific or random mutagenesis of ferredoxins, in particular Adx, are examined in view of the function of individual amino acids e.g. during electron transfer.

In yet another preferred embodiments libraries of variants of P450 enzymes or flavoproteins are examined in view of the role of defined amino acids during certain functions, in particular catalytical functions.

In general, these particular embodiments concern the production of variants of proteins and/or enzymes and the production of libraries with variants of proteins and/or enyzmes, respectively, which carry a prosthetic group or are coenzymes etc. or multimers etc. and which are screened in view of a certain characteristic, i.e. one or optionally several variants fulfilling this desired characteristic perfectly are selected. By selecting the variant the cell is selected, too, and carries the nucleic acid coding the variant. Thus, at the same time both the amino acid sequence and the structural information of the variant can be determined via the nucleic acid sequence. The characteristics in question are particularly enzyme inhibiting, catalytical, toxin degrading, synthesizing, therapeutical etc. characteristics.

Moreover, the host cell or the membrane preparation may be used as an assay system for a screening procedure, e.g. for identifying modulators (activators or inhibitors) of displayed polypeptides such as P450 enzymes or flavoproteins which may be used as potential therapeutic agents. The screening procedure may also be used to identify variants of displayed polypeptides having predetermined desired characteristics. For this purpose, libraries of modulators and/or libraries of displayed polypeptides may be used. Further, the host cells or membrane preparations derived therefrom may be used as a system for toxicity monitoring and/or degrading toxic substances in the environment, in the laboratory or in biological, e.g. human, animal, or non-biological systems.

An essential advantage of applying the host cells and membranes according to the invention is enabling correct folding and biological activity of proteins or protein complexes, e.g. P450 enzymes or flavoproteins, which require a membrane environment. Thus, a reconstitution as previously considered to be necessary is no longer required. Thereby the production steps of a functional biocatalytic-system are simplified and an increased stability of the system per se is obtained.

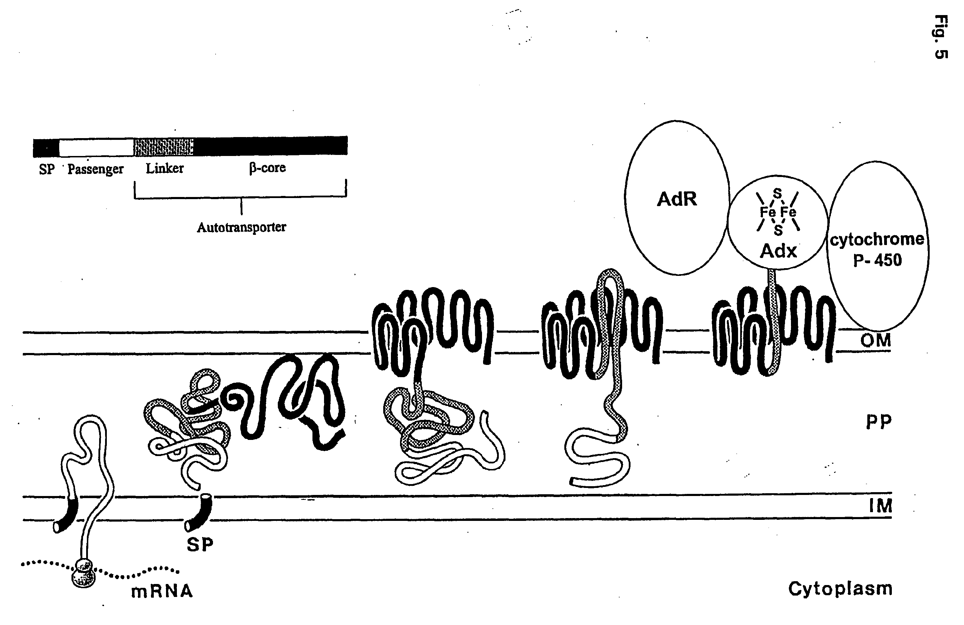

This is achieved in a particular embodiment by transporting the polypeptide chain of Adx to the surface with the help of an autotransporter, subsequently inserting the prosthetic group and adding AdR and P450 enzymes e.g. CYP11B1 or CYP11A1 externally. Thus, a functional complex is formed from AdR, Adx and P450 (Figure 5, Figure 9 and Figure 14), wherein the membrane environment is provided by the bacterial cell so that adding an artificial membrane or further detergents or a reconstitution as in previously used systems is not necessary. However, if desired, low amounts of detergent can be added to the host cell and/or membrane preparation according to the invention. These functional complexes can serve as carrier to target e.g. soil that need detoxification or can be used for the synthesis of steroids or plant or micororganism secondary metabolites. Further specific examples of previously reconstituted systems, which can be replaced by the system according to the invention described herein can e.g. be found in catalogues of the companies BD GENTEST, BD Biosciences, 6 Henshaw Street, Woburn, Massachusetts 01801 and PanVera Corporation, 545 Science Drive, Madison, WI 53711 U.S.A.

Moreover, the method according to the invention allows for an efficient expression of passenger proteins on the surface of host cells, particularly *E. coli* or other gram-negative bacterial cells up to 100 000 or more molecules per cell by using a liquid medium of the following composition: 5 g/l to 20 g/l, preferably about 10 g/l trypton, 2 g/l to 10 g/I, preferably about 5 g/l yeast extract, 5 g/l to 20 g/l, in particular about 10 g/l NaCl and the remaning part water. The medium should possibly contain as little as possible divalent cations, thus preferably Aqua bidest or highly purified water, e.g. Millipore water is used. The liquid medium contains in addition preferably EDTA in a concentration of 2 *µ*M to 20 *µ*M, in particular 10 *µ*M. Moreover, it contains preferably reducing reagents, such as 2-mercapto ethanol or dithiotreitol or dithioerythritol in a preferred concentration of 2 mM to 20 mM. The reducing reagents favour a non-folded structure of the polypeptide during transport. The liquid medium can further contain additional C-sources, preferably glucose, e.g. in an amount of up to 10 g/l, in order to favour secretion i.e. transfer of the passenger to the surrounding medium. For surface display preferably no additional C-source is added.

In a particulary preferred embodiment of the present invention host cells are provided, which carry a ferredoxin e.g. Adx or/and AdR or/and P450 reductase or/and cytochrome b5 or/and P450 enzymes or/and one of the peptides described in the following on their surface in a way that at least one polypeptide chain is brought to the surface with the help of an autotransporter and the prosthetic groups are inserted afterwards in replacement of previously used microsomal systems or systems reconstituted with the help of artificial or natural membranes or membrane parts.

Further preferred examples for the recombinant polypeptide to be displayed, i.e. the passenger polypeptides are peptides or proteins selected from the group of drug metabolizing enzymes, such as CYP1 A2 involved in the activation of aromatic amine carcinogenes, heterocyclic arylamine promutagenes derived from food pyrolysates and aflatoxin B1 (Gallagher EP, Wienkers LC, Stapleton PL, Kunze KL, Eaton DL., Role of human microsomal and human complementary DNA-expressed cytochromes P4501A2 and P4503A4 in the bioactivation of aflatoxin B1. Cancer Res. 1994, Jan 1;54(1):101-8) or CYP2E1 capable of activating the procarcinogenes N-nitrosodimethylamine and N-nitrosodiethylamin and metabolizes the procarcinogenes benzene, styrene, carbon tetrachloride, chloroform (Yoo JS, Ishazaki H, Yang CS., Roles of cytochrome P450IIE1 in the dealkylation and denitrosation of N-nitrosodimethylamine and N-nitrosodiethylamine in rat liver microsomes. Carcinogenesis. 1990 Dec; 11 (12):2239-43; Peter R, Bocker R, Beaune PH, Iwaskai M, Guengerich FP, Yang CS., Hydroxylation of chlorzoxazone as a specific probe for human liver cytochrome P-450IIE1. Chem. Res. Toxicol. 1990 Nov-Dec;3(6):566-73). Further preferred passenger peptides are peptides from the group of steroid biosynthesis enyzmes, such as CYP11 B1 involved in the formation of cortisol and aldosterone (Bernhardt R., Cytochrome P450: structure, function and generation of reactive oxygen species. Rev. Physiol. Biochem. Pharmacol. 1996;127:137-221) or CYP19 involved in the conversion of adrostenedione to 19-hydroxyandrostenedione, 19-oxo-androstenedione and estrone (Ryan KJ., Biological aromatization of steroids. J. Biol. Chem. 1959;134:268). Further examples for preferred passenger peptides are peptides from the group of metal ion containing enzymes, in particular Zn-containing enzymes, such as Zn-containing lactamases, carboanhydrase and alcohol dehydrogenase, Mg-containing enzymes, such as hexokinase, glucose-6-phosphatase or pyruvate kinase, Ca-containing enzymes, Fe-containing enzymes such as cytochrome oxidase, catalase, peroxidase or Ni-containing enzymes, such as urease catalyzing the hydrolization of urea to form ammonia and carbondioxide, or the hydrolization of urea-like compounds (Mobley HL, Island MD, Hausinger RP., Molecular biology of microbial ureases. Microbiol. Rev. 1995 Sep;59(3):451-80. Review; Soriano A, Colpas GJ, Hausinger RP., UreE stimulation of GTP-dependent urease activation in the UreD-UreF-UreG-urease apoprotein complex. Biochemistry. 2000 Oct 10;39(40):12435-40) or peptides described and supplied from BD Gentest, BD Biosciences, 6 Henshaw Street, Woburn, Massachusetts 01801 or from PanVera Corporation, 545 Science Drive, Madison, WI 537111 U.S.A. Further preferred metal ion containing enzymes are Cu-containing enzymes, such as cytochrome-oxidase, Mn-containing enzymes, such as arginase and ribonucleotide reductase, Mo-containing enzymes, such as dinitrogenase and Se-containing enzymes, such as glutathione peroxidase.

Further preferred examples of the recombinant polypeptide to be displayed, i.e. the passenger polypeptides are peptides or proteins from the group of flavoproteins, e.g. oxidoreductases, dehydrogenases or oxidases, in particular sugar oxidases, such as pyranose oxidase. Thus, for example, host cells are provided, which carry a sugar oxidase, e.g. pyranose oxidase, on their surface by bringing the polypeptide chain of the sugar oxidase to the surface with the help of an autotransporter and subsequently adding the prosthetic group FAD. Preferably, this can be achieved by adding purified FAD in a buffer solution to the host cells displaying the sugar oxidase polypeptide chain, wherein the buffer solution contains FAD in excess, 1 mM dithiothreitol, 0.1 mM EDTA, 17% glycerol (v/v), 0.1 M guanidine/HCl and 0.1 M HEPES, pH 7.5. In this connection it should be noted that the procedure described above for the insertion of the prosthetic group can also be transferred to those flavoproteins that are described to be homotetramers, homodimers, etc. The host cells or membrane preparations derived therefrom may be used as systems for the synthesis of sugars, building blocks, fine chemicals, basic chemicals and chiral compounds.

In a further preferred embodiment of the present invention host cells and/or membrane preparations are provided, which carry at least one P450 enzyme, on their surface in a way that at least one polypeptide chain is brought to the surface with the help of an autotransporter and the prosthetic groups are inserted afterwards in replacement of previously used microsomal systems or systems reconstituted with the help of artificial or natural membranes or membrane parts. Preferably the P450 enzymes are hepatic P450 enzymes, particularly P450 3A4, 2D6, 2C9 and 2C19. The host cells and/or preparations according to the invention are preferably used sequentially for testing the enzyme inhibition of P450 enzymes. For example, with the help of the host cell and/or membrane preparation according to the invention it can be found out in an early step of drug discovery, the so-called lead identification, whether the new drug lead structure to be tested could possibly have side-effects or lead to the so-called drug-drug interaction.

Further, the present invention shall be further illustrated by the following figures and examples:
- Figure 1:: Adx dependent reactions of CYP11A1 and CYP11B1. The electron transfer activity of surface-presented Adx was analyzed in reconstituted systems containing the natural final electron acceptors CYP11A1 and CYP11B1 catalyzing the indicated chemical reactions.
- Figure 2:: (A) Nucleotide and amino acid sequence of bovine adrenodoxin, devoid of the mitochondrial target sequence as used in this study.
(B) Structure of the fusion protein FP66. The environment of the fusion sites are given as sequences. Signal peptidase and trypsin cleavage sites are indicated.
- Figure 3:: SDS-PAGE (A) and Western blot analysis (B) of outer membrane preparations from *E. coli* UT5600 (lanes 1, 2) and UT5600 pJJ004 (3,4,5,6). Conditions and molecular weight markers as indicated. *signals that correspond to fusion proteins multimers. C1 = core 1; C2 = core 2.

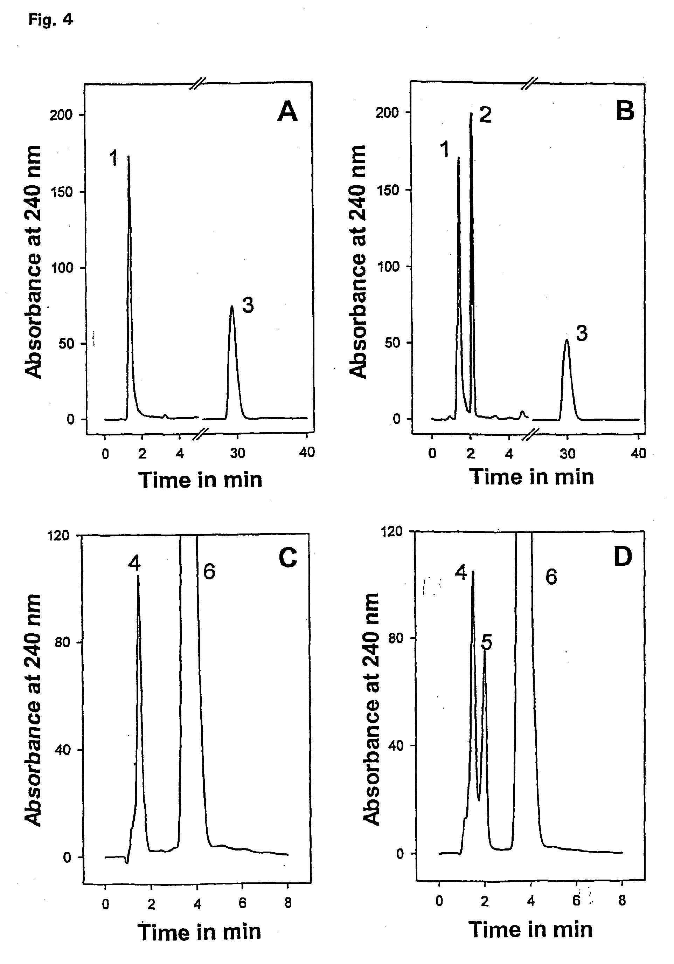

- Figure 4:: HPLC chromatograms of CYP11A1 and CYP11B1-dependent substrate conversion. Chromatograms were obtained from extracted samples with *E. coli* cells containing pAT-Adx04 before reconstitution (A, C) and after reconstitution (B, D) of the iron-sulfur cluster in surface displayed adrenodoxin. The indicated substance peaks in CYP11A1 reactions (A, B) represent 1) cortisol (internal standard), 2) pregnenolone (product) and 3) cholesterone (substrate), in CYP11B1 reactions (C, D) 4) cortisol (internal standard), 5) corticosterone (product) and 6) deoxycorticosterone (substrate). Steroids were analyzed for A and B with an isocratic solvent system of acetonitril/isopropanol (15: 1) and for B and C with an isocratic solvent system of 50% acetonitril.
- Figure 5:: Schematic representation of Adx surface display by the autotransporter pathway in *E. coli.*
- Figure 6:: SDS-Page (A) and Western blot (B) of outer membrane preparations from *E. coli* UT2300 (lane 1) and *E. coli* UT2300 pJJ004 (lane 2). Before outer membrane were prepared, whole cells were digested with trypsin (+) or not (-). Western blot was performed with an Adx specific antibody.
- Figure 7:: Western blot of outer membrane preparations from *E. coli* UT2300 pJJ004 grown in different culture media. red. = reducing conditions in culture medium, gluc. = 5 g/l glucose, t.e. = trace elements in culture medium. Western blot was performed with an Adx specific antibody.
- Figure 8:: Western blot of outer membrane preparations from *E. coli* UT2300 pJJ004 treated with different sample buffers before SDS-Gel separation. SB +: sample buffer contained mercaptoethanol, red -: sample buffer without mercaptoethanol. Western blot was performed with an Adx specific antibody.
- Figure 9:: Schematic representation of functional ADX dimers on the surface of *E. coli.*
- Figure 10:: SDS-PAGE (A) and Western blot analysis of outer membrane preparations from *E. coli* UT5600 pJJ004. Whole cells were either digested with trypsin (lane 2) or not (1) before outer membranes were prepared. Before being applied to SDS-PAGE samples were boiled in sample buffer without 2-mercaptoethanol. The sizes of the molecular weight marker bands are indicated. Natural outer membrane proteins OmpF/C and OmpA are marked. The Adx specific antibody used for detection has been described previously (35).
- Figure 11:: (A) Western blot analysis of supernatant proteins from OmpT⁺ *E. coli* UT2300. Before applied to SDS-PAGE samples were boiled with (lane 1) or without 2-mercaptoethanol (2).The sizes of the molecular weight marker proteins are indicated.
(B) Molecular weight determination of free, recombinant Adx. The size of purified Adx molecules, not connected to the autotransporter domains was determined by the use of size exclusion chromatography. The proteins indicated in the plot (circles with grey fill color) were used to generate a standard curve. The retention volume of Adx is shown as a circle with white fill color and the extrapolated MW is given in brackets.

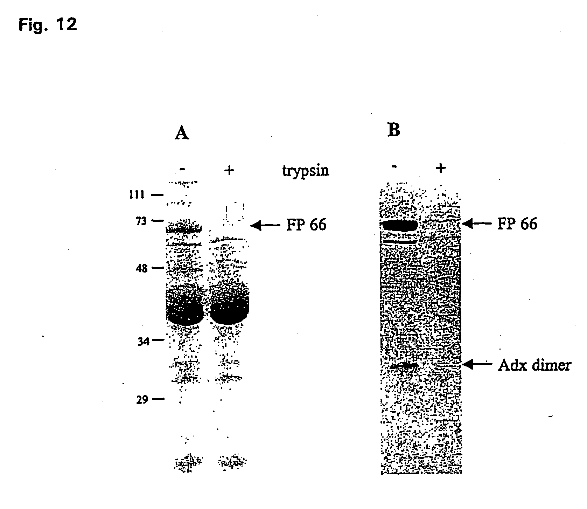

- Figure 12:: Released dimeric Adx on the surface of *E. coli* UT2300 pJJ004. Before outer membrane proteins were prepared as described in Example 3 and subjected to SDS-PAGE (A) and Western blot analysis (B), whole cells were treated with trypsin (+) or not (-). For sample preparation, buffer without 2-mercaptoethanol was used. The migration of the molecular weight maker proteins is indicated in kilodaltons.

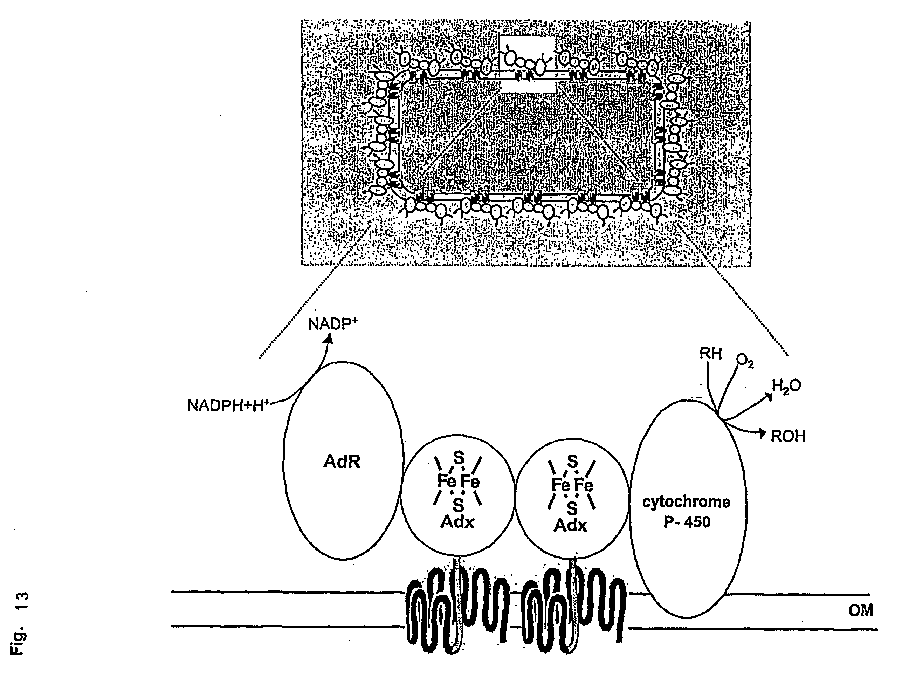

- Figure 13:: Schematic view of the whole cell steroid conversion by autotransporter mediated surface display of dimeric Adx in *E. coli.*

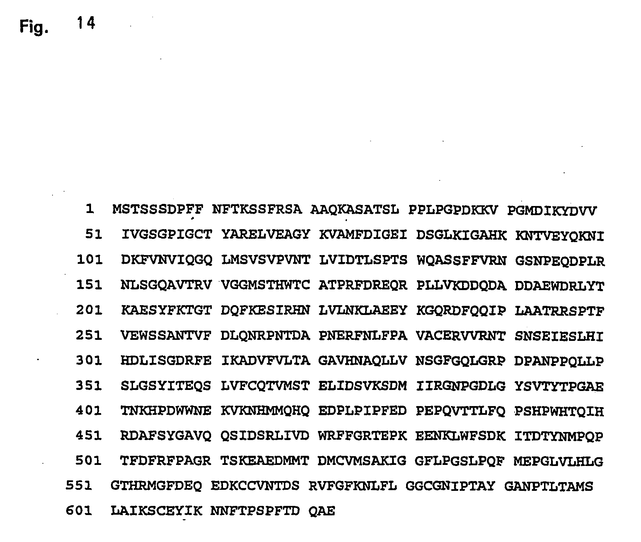

- Figure 14:: Amino acid sequence of pyranose oxidase from Coriolusolor as can be used in this study.

### Example 1

Functional surface display of a bovine [2Fe-2S] ferredoxin by the autotransporter pathway in *E. coli.*

### 1.1 Experimental protocol

### Bacterial strains, plasmids and culture conditions

*E. coli* UT5600 (F⁻ *ara 14 leuB6 azi-6 lacY1 proC14 tsx-67 entA403 trpE38 rfbD1 rpsL109 xyl-5 mtl*-*1 thi1,* Δ*ompT - fepC266*) was used for the expression of autotransporter fusion proteins (42). *E. coli* TOP1 0 (F^{*-*} *mcrA* Δ*(mrr-hsdRMS-mcrBC) φ80lacZΔM15 ΔlacX74 deoR recA1 araD139* Δ*(ara-leu) 7697 galU galK rpsL (Str*^{*R*}*) endA1 nupG*) and the vector pTOPO10, used for subcloning of PCR products, were obtained from Invitrogen (Groningen, the Netherlands). Plasmid pJM007 (15), encoding the AIDA-I autotransporter and plasmid pKKHCAdx (43), encoding bovine adrenodoxin have been described elsewhere. Bacteria were routinely grown at 37°C in Luria-Bertani (LB) broth, containing 100 mg of ampicillin liter⁻¹. For Adx expression studies, EDTA was added to a final concentration of 10 *µ*M and β-mercaptoethanol was added to a final concentration of 10 mM.

### Recombinant DNA techniques

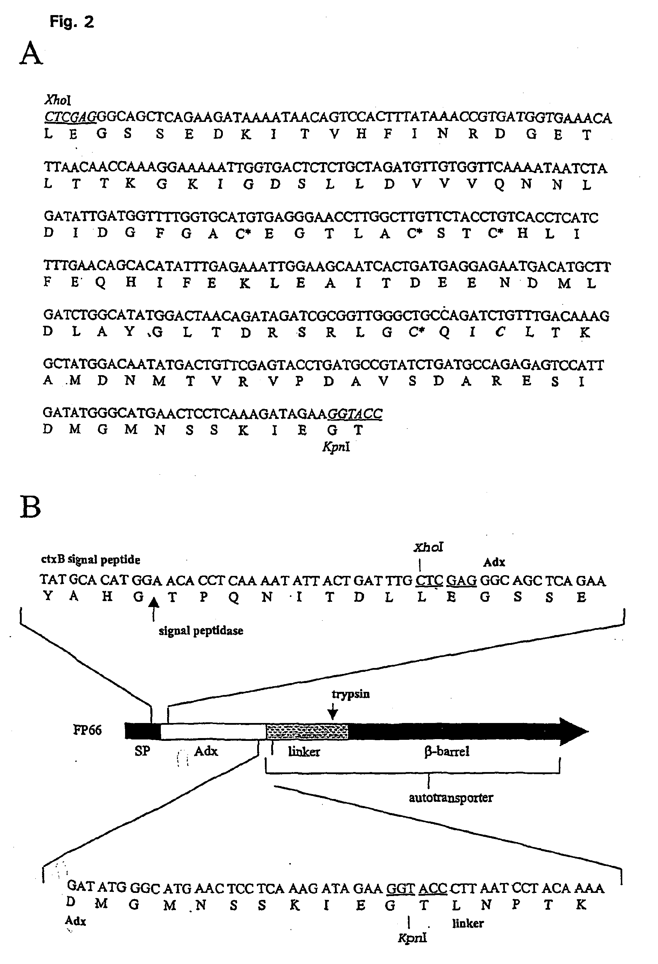

For the construction of the Adx-autotransporter fusion, the Adx gene was amplified by PCR from plasmid pKKHCAdx using oligonucleotide primers JJ3 (5'-ccgctcgagggcagctcagaagataaaataacagtc-3') and JJ4 (5'-ggggtaccttctatctttgaggagttcatg-3'). The PCR product was inserted into vector pTOP010 and recleaved with *Xho*I and *Kpn*I*.* The restriction fragment was ligated to pJM7, restricted with the same enzymes. This yields an in-frame fusion of Adx with the AIDA-I autotransporter, under the control of the strong P_{TK} promoter (Fig 2b).

### Outer membrane preparation

*E. coli* cells were grown overnight and 1 ml of the overnight culture was used to inoculate 20 ml LB medium. Cells were cultured at 37°C with vigorous shaking (200 rpm) for about 5 h until an OD₅₇₈ of 0,7 was reached. After harvesting and washing with phosphate-buffered saline (PBS), outer membranes were prepared according to the rapid isolation method of Hantke (44). For whole cell protease-treatment, *E. coli* cells were harvested, washed and resuspended in 5 ml PBS. Trypsin was added to a final concentration of 50 mg liter ⁻¹ and cells were incubated for 5 min at 37°C. Digestion was stopped by washing the cells three times with PBS containing 10% fetal calf serum (FCS) and outer membranes were prepared as described above.

### SDS-Page and Western blot analysis

Outer membrane isolates were diluted 1:2 with 2 x sample buffer (100 mM Tris/HCl, pH 6.8, 4% SDS, 0.2% bromphenol blue, 20% glycerol), either with (reducing) or without 2-mercaptoethanol (non-reducing conditions), boiled for 20 min and analyzed on 12.5% SDS-PAGE. Proteins were visualized with Coomassie brilliant blue with prestained molecular weight protein markers (Bio-Rad, München, Germany). For Western blot analysis, gels were electroblotted onto polyvinylidene-difluoride (PVDF) membranes and blotted membranes were blocked in PBS with 3% FCS overnight. For immunodetection, membranes were incubated with primary anti-Adx antibody, diluted 1:500 in PBS with 3% FCS for 3 hours. Prior to addition of the secondary antibody, immunoblots were rinsed three times with PBS. Antigen-antibody conjugates were visualized by reaction with horseradish peroxidase-linked goat anti-rabbit IgG secondary antibody (Sigma, Deisenhofen, Germany), dilutes 1:1000 in PBS. Color reaction was achieved by adding a solution consisting of 2 ml 4-chlor-1-naphtol (3 mg/ml ethanol), 25 ml PBS and 10 µl H₂O₂ (30%).

### Reconstitution of the [2Fe-2S] Center in Adrenodoxin

Cells were grown overnight, washed two times in PBS and resuspended to a calculated final OD₅₇₈ of 50. Refolding of adrenodoxin on the surface of the *E*. *coli* cells was achieved either after heat denaturation at 70°C, 60°C, and 40°C followed by a slow temperature ramp to 22°C, or was performed directly at ambient temperature. Simultaneous chemical reconstitution of the iron-sulfur cluster was performed under strictly anaerobic conditions in 50 mM Tris•Cl buffer (pH 7.4). The bacterial suspension (4 ml) was supplemented with 1 mM β-mercaptoethanol and 0.2 mM ferrous ammonium sulfate and was slowly titrated with 100 ml of a solution containing 100 mM Li₂S and 2 mM DTT (45).

### Protein Purification

Recombinant adrenodoxin (Adx) and adrenodoxin reductase (AdR) were purified as described (32,46). Protein concentration was calculated using ε₄₁₄ = 9.8 (mM cm)⁻¹ for Adx (47) and ε₄₅₀ =11.3 (mM cm)⁻¹ for AdR (48). Isolation of CYP11A1 and CYP11B1 from bovine adrenals was performed according to Akhrem et al. (49) with slight modifications.

### Enzyme Activity Assays

The biological electron transfer function of adrenodoxin was detected in adrenodoxin-dependent reactions containing its natural effector enzymes, cytochromes CYP11A1 and CYP11B1. The cholesterol side chain cleavage activity of cytochrome CYP11A1 was assayed in a reconstituted system catalyzing the conversion of cholesterol to pregnenolone. Assays were performed at 37°C in 50 mM potassium phosphate (pH 7.4), 0.1% Tween 20 and contained 100 µl *E. coli* cells, 0.5 µM adrenodoxin reductase, 0.4 µM CYP11A1, 400 *µ*M cholesterol and a NADPH regenerating system. After the reaction, the steroids were converted into their corresponding 3-one-4en forms by the addition of 2 units/ml cholesterol oxidase, extracted, and analyzed by reversed phase HPLC. Substrate conversion from deoxycorticosterone to corticosterone in cytochrome CYP11B1 assays were performed at 37°C in 50 mM potassium phosphate (pH 7.4), 0.1 % Tween 20 and consisted of 100 *µ*l *E. coli* cells, 0.5 *µ*M adrenodoxin reductase, 0.2 *µ*M CYP11 B1, 400 *µ*M deoxycorticosterone and a NADPH regenerating system. Extracted steroids were separated on a Jasco reversed phase HPLC System of the LC800 Series using a 3.9 x 150 mm Waters Nova-Pak C₁₈ column. The amounts of reconstituted Adx on the cell surface was estimated by comparison of the steroids produced in cell-dependent reactions with reactions which contained defined amounts of purified holo-Adx.

### Electron Spin Resonance Measurements

EPR measurements were carried out on a Bruker ESP300E spectrometer at -163°C. Cell samples in 50 mM potassium phosphate (pH 7.4) were dithionite-reduced under anaerobic conditions and frozen in liquid nitrogen.

### 1.2 Results

### Fusion protein construction

To obtain an in frame fusion with the gene segments needed for autodisplay, the coding region of bovine adrenodoxin (Adx) was PCR amplified. The PCR primers used added a *Xho*I site at the 5' end and a *Kpn*I site at the 3' end of the Adx encoding region. To avoid any hindrance with bacterial surface translocation, an Adx gene was used as PCR template that was devoid of the mitochondrial targeting sequences (32). The amino acid and the nucleotide sequence of the resulting PCR product, which was confirmed by dideoxy sequencing is shown in Fig. 2a. For the construction of the recombinant fusion protein-encoding gene, plasmid pJM7 was cleaved with *Xho*I and *Kpn*I*.* pJM7 is a pBR322-derived high copy number plasmid that directs the expression of a choleratoxin β-AIDA-I fusion protein under control of the constitutive P_{TK} promoter (15,23). Cleavage with *Xho*I and *Kpn*I resulted in the deletion of the choleratoxin β (CTB) encoding DNA region. Insertion of the cleaved Adx PCR fragment yielded plasmid pJJ004, which encoded a fusion protein consisting of the signal peptide of CTB, Adx, and the AIDA-I autotransporter region, including a linker region, which proved to be sufficient for full surface access (Fig. 2b). Due to the ligation procedure the artificial construct still contains seven amino acids of mature CTB. Based on the predicted molecular mass of 65.9 kDa, the fusion protein was termed FP66. Export of FP66 was investigated in *E. coli.*

### Probing the surface display of bovine Adx

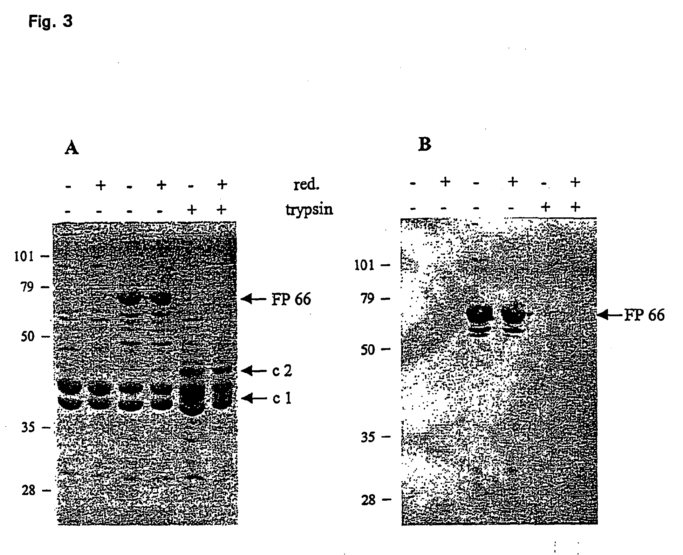

Most of the available *E. coli* host strains possess an outer membrane protease (OmpT) that catalyzes the sequence specific release of surface-exposed proteins (33). As the linker used in our Adx-AIDAβ fusion contains an OmpT protease specific cleavage site, it was necessary to use an *omp*T-negative strain for Adx surface display. In former studies *E. coli* UT5600 (ompT) proved to be suitable to prevent cleavage of surface-exposed autotransporter fusion proteins (23,34). Therefore pJJ004 was transformed into *E. coli* UT5600 and the expression of FP66 was monitored by SDS-PAGE and immunoblotting of outer membrane protein preparations. As shown in Fig 3a, FP66 could be easily detected by Coomassie brilliant blue staining of outer membrane proteins. Expression was almost at the same level as expression of the natural outer membrane proteins OmpA and OmpF/C. Neither growth rate nor optical density reached in the stationary phase of *E. coli* UT5600 grown in liquid medium was decreased by the expression of FP66 (not shown). Electrophoretic mobility of the Adx-AIDAβ-fusion protein was in perfect agreement with the predicted molecular mass of 65.9 kDa. Finally, the identity of FP66 was confirmed by Western blot analysis using an Adx specific polyclonal rabbit antibody (35) (Fig. 3b). The application of non-reducing conditions resulted in faint bands at molecular weights that corresponded to multimers of the fusion protein.

To clarify whether the passenger domain of FP66 was accessible at the surface and not directed to the periplasma, intact cells of *E. coli* UT5600 pJJ004 were subjected to protease digestion with trypsin. Outer membranes were subsequently prepared from these cells and analyzed by SDS-PAGE and Western blotting. In former studies it has been shown that membrane embedding of the AIDA-I autotransporter resulted in a trypsin resistant core of 37.1 kDa (15). This core is due to a trypsin cleavage site found in the linker region (Fig. 2b). Predicted trypsin cleavage sites that are located closer to the C-terminus of the transporter are protected from trypsin access by membrane topology. As can bee seen by SDS-PAGE and subsequent staining with Coomassie blue, external trypsin addition resulted in the disappearance of the full-size-fusion protein and generated two lower-molecular-weight products (Fig. 3a). One of them corresponds to the 37.1 kDa trypsin resistant autotransporter core. The second digestion product (core 2) has a larger molecular weight of around 45 kDa. Therefore it must result from trypsin cleavage within the Adx passenger domain. Folding subsequent to processing could hinder trypsin access to the linker region and therefore prevent further degradation. Bovine Adx is known to contain several consensus trypsin cleavage sites. Obviously in our experiments, there was a preference for the trypsin cleavage of distinct sequence, as beside the 45 kDa core 2, there was no other prominent digestion product detectable.

Electron transfer function of bovine Adx displayed on the bacterial surface As the previous results clearly indicated that bovine adrenodoxin (Adx) is transported to the bacterial surface by the autotransporter pathway, the next step was to investigate whether the biomolecule displayed is active. The activity was measured with the CYP11A1-dependent conversion of cholesterol to pregnenolone and with the CYP11B1-dependent conversion of deoxycorticosterone to corticosterone (Fig. 1). Both assays containing the native electron acceptors of Adx and whole cells of *E. coli* UT5600 pJJ004 showed no substrate conversion (Fig. 4). This emphasized that a self-assembled holo-Adx containing the redox-active [2Fe-2S] cluster is not formed after expression and transport to the cell surface. The absence of the iron-sulfur cluster was confirmed by EPR measurements with whole cells (not shown). This finding fits the concept of the autotransporter secretion mechanism published earlier (17,21). Accordingly, proteins can only be transported by the autotransporter pathway as long as they maintain a relaxed, unfolded confirmation. The *E. coli* ferredoxin, which is a structural homologue of adrenodoxin, obtains the [2Fe-S] cluster in the cytoplasm (36). We were not able, however, to detect any Adx-autotransporter fusion proteins in the cytoplasmic fraction of recombinant *E. coli* cells. This might indicate that signal peptide targeting prevents cluster incorporation or that cluster incorporation and transport intersection results in rapid proteolytic degradation in the cytoplasm. A periplasmic intermediate was also not detectable.

As the expression of apo-Adx on the *E. coli* surface was very effective it seemed worthwhile to assess post-transport integration of the [2Fe-2S] cluster into surface-displayed Adx by chemical reconstitution (37). Therefore, whole cells of *E. coli* UT5600 pJJ004 were incubated at different temperatures to verify optimal conditions for unfolding of Adx molecules displayed on the surface. Subsequently ferrous iron and thiols were added under anaerobic conditions, either in a nitrogen or in an argon atmosphere. The following extremely slow dilution of the bacterial suspension by Li₂S resulted in low overall sulfide concentrations and avoided precipitation of iron sulfides (38). Moreover, it allowed the successful refolding of the Adx peptide around a [2Fe-2S] cluster. This was indicated by a significant product formation in both cytochrome P450 tests, when whole cells where added after the reconstitution procedure (Fig. 4). As controls were negative and no additional Adx was supplied, this must clearly be due to an electron transfer function of surface-displayed Adx (Fig. 4), reporting successful chemical reconstitution. From these results it can be concluded that Adx displayed on the *E. coli* surface by the autotransporter pathway is biologically active and can transfer electrons to the P450 enzymes CYP11A1 and CYP11B1. The number of active Adx molecules after reconstitution on the surface of *E. coli* could be estimated by the use of the specific substrate conversion rate in the CYP11A1 assay. For this purpose the enzyme assay was performed without *E. coli* cells but with different concentrations of purified holo-Adx.

This enabled to record a calibration curve, describing substrate conversion in dependence of Adx molecules in the assay. The calibration curve was used to estimate the apparent number of active Adx molecules of *E. coli* UT5600 pJJ004 under assay conditions. As can be seen in Table 1 reconstitution of the [2Fe-2S] cluster was successful at all temperature conditions applied. It seemed to be favored at the lower temperatures of 22 and 40°C, whereas heat denaturation at 60°C or 70°C prior to refolding experiments resulted in significant decreased amounts of assembled holo-Adx. Moreover, denaturation conditions of 70°C dramatically decreased the number of viable bacterial cells. An apparent number of 1.8 x 10⁵ functional Adx molecules per cell indicated optimal conditions for refolding of the peptide around the [2Fe-2S] center at 22°C. A preceding heat denaturation of surface-displayed apo-Adx was obviously not necessary for successful cluster incorporation.

### 1.3 Discussion

In the present study, bovine adrenodoxin was expressed on the *E. coli* cell surface by the autotransporter pathway. The expression rate was in the same order of magnitude as the expression of natural outer membrane proteins OmpF/C or OmpA without disturbing outer membrane integrity or reducing cell growth (Fig. 3a). Adx passenger molecules transported to the cell surface by the autotransporter initially did not contain an iron-sulfur cluster. This fits the concept of the autotransporter secretion mechanism (15,17,20). Accordingly, the C-terminus of this secreted proteins forms a porin-like structure, a so-called β-barrel in the outer membrane and proteins with stable and extended three dimensional structures cannot pass this gate. Incorporation of the [2Fe-2S] cluster into apo-Adx results in the acquisition of a stable structure (29) and is therefore not compatible to surface translocation. Apo-Adx expressed on the *E. coli* cell surface could be supplemented with the [2Fe-2S] cluster by a one-step reconstitution procedure under anaerobic conditions. Anaerobic conditions proved to be best in an argon atmosphere (not shown). Reconstitution resulted in viable cells that expressed biologically active Adx on the surface. Whole cells expressing Adx could be efficiently used to transfer electrons from adrenodoxin reductase to P450 enzymes CYP11A1 and CYP11B1 (Fig. 4). Activity could easily be quantified by determining Adx depending product formation of either pregnenolone or corticosterone by HPLC. By calibrating Adx activity in the substrate formation assay by purified holo-Adx, the apparent number of active Adx molecules could be determined as more than 100.000 molecules/cell. The high copy number of recombinant outer membrane proteins is at the same level as it has been reported for natural *E. coli* outer membrane protein OmpA (39). High expression of recombinant and chemically reconstituted, active Adx, had no influence on the viability of *E. coli.* Reconstitution at higher temperature in order to get easier unfolding of the Apo-Adx peptide chain did not result in a better [2Fe-2S] cluster incorporation. Moreover, higher temperatures significantly decreased the number of viable cells. The best ratio of active Adx molecules expressed on the surface and viable *E. coli* cells was obtained at room temperature conditions (Table 1). This indicates that apo-Adx, displayed on the surface, forms a structure well prepared for efficient [2Fe-2S] cluster acceptance, even at non-denaturating conditions.

Our results show that the autotransporter pathway can be used for the surface display of proteins that contain an inorganic prosthetic group. The catalytic activity of the reconstituted Adx indicates that it is freely accessible at the cell surface, even for molecules as large as AdR or P450 enzymes, as there must be direct contact between Adx and AdR or P450, respectively, to yield electron transfer (40) (Fig. 5). The expression of more than 100.000 active molecules per cell without hampering cell viability is to our knowledge unique among bacterial surface display systems applied so far. It enables to provide high Adx activity by whole cells as carriers or protectors. This has promising options for further applications. On one hand, the role of distinct amino acids in the electron transfer through Adx or in the interaction with its redox partners can be studied either by random or by rational variation of the protein, without the need of mutant enzyme purification. On the other hand, the system developed here - efficient surface translocation of an unfolded apo-protein and chemical reconstitution of the prostethic group - is also be applicable to proteins containing FAD, FMN or heme groups. Especially heme containing P450 enzymes are important, as they are involved in the syntheses of a wide variety of valuable products but also in the degradation of numerous toxic compounds (41). With intent to use enzyme-coated cells for these applications, autotransporter mediated surface display of e.g. P450 enzymes could open a new dimension in developing whole cell factories.

### Example 2

### Dimer formation of Adx molecules on the surface of E. coli

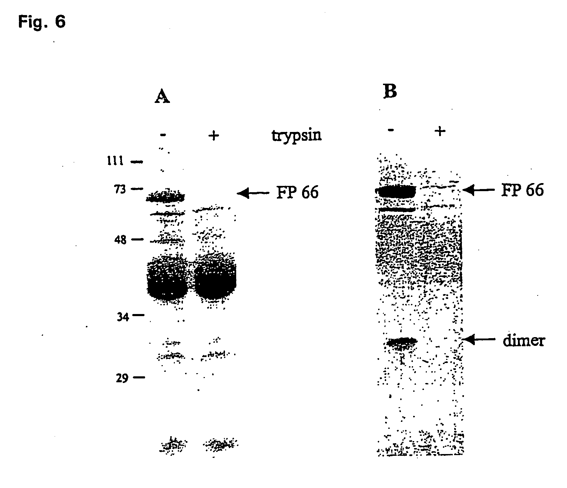

The SDS-PAGE results indicated that the passenger domain of FP66 is located at the bacterial surface. This could be supported by Western blotting. Adx was only detected in the outer membrane of undigested *E. coli* cells, while digestion with trypsin caused degradation of the immunoreactive domain in the Adx-autotransporter fusion protein. This indicated surface accessibility of the N terminus of FP66. From this point of view, it was interesting to see, whether the surface-exposed Adx could be released into the supernatant. For that purpose plasmid pJJ004 was propagated in *E. coli* UT2300 (42). *E. coli* UT2300 is an isogenic strain of *E. coli* UT5600, with the exception that it expresses the outer membrane protease OmpT. In earlier studies it has been shown that OmpT cleaves autotransporter-fusion proteins at the bacterial surface, resulting in the release of an intact passenger (15,23,34). In our experiments, however, we were not able to detect any Adx in the supernatants of *E. coli* UT2300 expressing the fusion protein. To elucidate the fate of the passenger in this strain, outer membranes were prepared and subjected to SDS-PAGE and subsequent Western blot analysis. By this means it turned out that OmpT was not completely able to cleave the Adx-autotransporter fusion protein. There was still a substantial amount of full size fusion protein detectable in the outer membrane of *E. coli* UT2300 pJJ004 (Fig. 6), although to a weaker extent as in *E. coli* UT5600. Moreover an additional band appeared, corresponding to a protein with an apparent molecular mass of about 33 kDa, which was recognized by the Adx specific antiserum. This can be explained by the formation of dimers by the Adx passenger portion of the fusion proteins, which are released from the autotransporter by OmpT cleavage, but still remain associated with the outer membrane. The slightly increased apparent molecular weight (33 kDa) in comparison to the molecular weight of native Adx dimers (28 Kda) seems to be due to a part of the linker region, which remained connected to the Adx passenger due to OmpT processing (Fig. 1). More recently the determination of the crystal structure revealed that functional Adx is a dimer (50). This has been proposed earlier by the mechanism of P450-dependant electron transfer, but it is still matter of discussion at current, whether functional Adx is a dimer or a monomer (31,37).

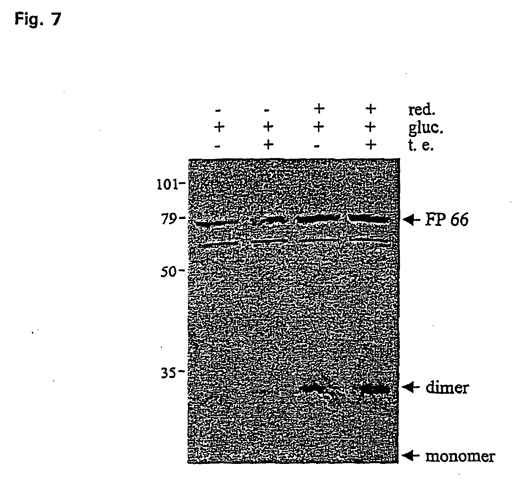

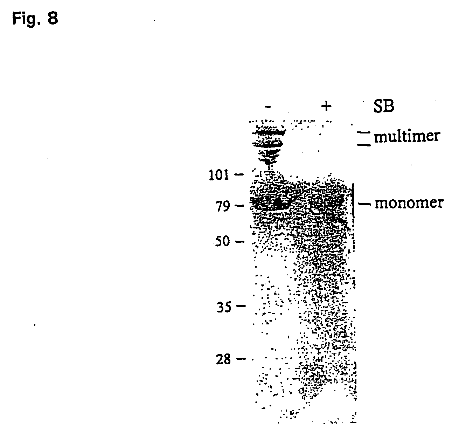

It is not yet clear why the Adx dimers cleaved off from the transporter domain remained associated to the outer membrane and were not released to the supernatant. It has been shown earlier that Adx not only forms dimers but also forms tetramers. By this view it might be possible that Adx dimers that were released by OmpT form hybrid tetramers with Adx molecules that are still connected to the surface by the autotransporter domain. It cannot be excluded that some amount of Adx was released to the supernatant, but remained below the detection limit. At this point it seems worth emphazising that incomplete processing of autotransporter proteins by OmpT is not common. It might indeed be a result of a reduced protease access due to a folding influence of the Adx passenger or even due to Adx dimer or multimer formation. By supplementation of the growth medium with metal ions or other compounds, we did also not obtain complete cleavage of the Adx-autotransporter fusion by OmpT (Fig. 7). The hypothesis of multimer formation is supported by results that were obtained with *omp*T-negative *E. coli* UT5600 expressing FP66 analyzed by Western blotting. Under non-reducing condition, multimers of the the full-size-fusion protein appeared, indicating that the interaction between Adx passenger molecules is strong enough to keep several transporter domains together (Fig. 8). A protein band with an apparent molecular weight corresponding to a tetramer of free Adx molecules, however, could not be ascertained. There was a protein band of the correct molecular weight range in outer membrane preparations of *E. coli* UT2300 expressing the Adx-autotransporter fusion. But this band also appeared in UT5600 expressing the Adx-autotransporter fusion. Therefore it cannot be excluded that this is due to incomplete unfolding of the β-barrel (21), resulting in an increased electrophoretic mobility or to any unspecific cross-reactivity. External addition of trypsin resulted in the complete disappearence of the full-size-fusion protein as well as the putative Adx dimer (Fig. 6), underlining the surface location of both forms of the protein.

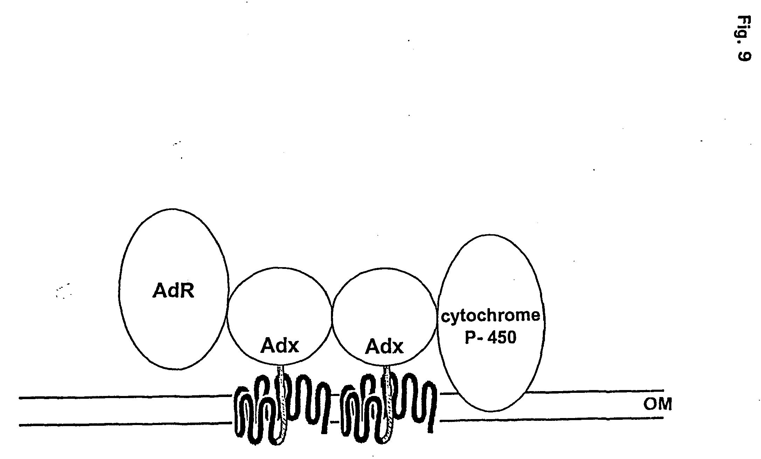

Our results indicate that passenger proteins displayed on the surface of *E. coli* (or any other gram negative bacterium) by the autotransporter pathway can form functional dimers or multimers. The affinity of the passenger domains can be sufficient to guide the assocciation of multiple transporter domains. Due to their amphipatic β-barrel structure, it seems reasonable that the transporter can move freely in the horizontal of the membrane. The data presented here are the first to provide experimental evidence. This opens the door to the application of autodisplay on enzymes or other proteins that are functionally dimers or multimers, even if their subunits are heterogenic. In this case, the heterogenic monomers may be expressed individually as fusions to the autotransporter domains simultaneously in one cell. The affinity of the subunits will guide their association at the surface, and will not be hampered by an restricted mobility of the membrane anchored β-barrel (Fig. 9).

At this point it is worth mentioning that the simple addition of Adr and P450 CYP11B1 or CYP11A1 to cells displaying Adx with the prosthetic group resulted in enzymatic activity. The addition of an artificial membrane or detergents as was necessary before, when free Adx was used instead of Adx displayed on the *E. coli* cell surface was not required. This indicates that the membrane environment, which is needed by P450 enzymes as CYP11B1 or CYP11A1 to fold into an active conformation, is provided by the bacterial surface.

The association of free Adx dimers to the outer membrane can also be explained by a permanent tendency of monomeric Adx to form dimers and of dimeric Adx molecules to split into monomers. As a consequence OmpT released Adx monomers could form dimers with Adx molecules, which are anchored to the membrane by the autotransporter portion. Possibly dimer formation of Adx-autotransporter fusion proteins, due to interaction of the passenger domain, hinders OmpT cleavage. Monomerization of the Adx-autotransporter fusion protein permits OmpT cleavage again leading to free Adx monomers, which can form dimers either with released or membrane-anchored Adx molecules. The permanent and rapid transformation from momomers to dimers and *vice versa* could result in a higher tendency of released Adx molecules to remain assocciated with the outer membrane. In this case either dimers or monomers could be found on the surface. Indeed, in some of the experiments, we were able to detect immunoreactive protein bands in the size of monomeric Adx by the Adx-specific antibody (Fig. 7).

### Example 3

### Cellular surface display of dimeric Adx and whole cell P450-mediated steroid synthesis on E.coli

### 3.1 Experimental protocol

### Plasmids, bacterial strains and conditions

Construction of plasmid pJJ004, which encodes a fusion of bovine Adx and the AIDA-I autotransporter genes has been described in Example 1. Plasmid pJJ004 was propagated in *E. coli* strains UT5600 (F⁻ *ara 14 leuB6 azi-6 lacYI proC14 tsx-67 entA403 trpE38 rfbDI rpsLI09 xyl-5 mt1-I thi1,* Δ*ompT- fepC266)* and UT2300 (F⁻ ara14 leuB6 azi-6 lacYI proC14 tsx-67 entA403 trpE38 rfbDI rpsLI09 xyl-5 mtl-I thil) (42). *E. coli* UT2300 releases surface-exposed passenger proteins, whereas *E. coli* UT5600 is not able to form the outer membrane protease T (OmpT), resulting in the surface display of passenger proteins that are translocated by the autotransporter pathway (15). Cells were routinely grown at 37°C in Luria-Bertani (LB) broth containing 100 mg I⁻¹ ampicillin, 10 µM EDTA and 10 mM 2-mercaptoethanol. For OmpT expression studies, glucose was added up to 5% and to achieve a real low metal ion supplementation, growth medium was prepared with tap water, when indicated.

### Isolation of outer membrane and supernatant proteins

*E.* coli cells were grown overnight in LB medium and 1 ml was used to inoculate a 20 ml culture. Cultivation was at 37°C with vigorous shaking (200 rpm) until an OD₅₇₈ of 0.7 was reached (mid log phase). Cells were harvested by centrifugation and after washing with phosphate-buffered saline (PBS), outer membranes were prepared according to the rapid isolation method of Hantke (44). For whole cell protease-treatment, *E. coli* cells were harvested, washed and resuspended in 5 ml PBS. Trypsin was added to a final concentration of 50 mg I⁻¹ and cells were incubated for 5 min at 37°C. Digestion was stopped by washing the cells three times in PBS containing 10% fetal calf serum (FCS) and outer membranes were prepared as described above. For supernatant protein preparation, a 20 ml culture of *E. coli* UT2300 pJJ004 was inoculated with 1 ml of an overnight culture and grown to the mid log phase. Cells were harvested, washed once and resuspended in 4 ml 10 mM Tris/HCl pH 8,0 for 5 min. After centrifugation, 20 *µ*l of 100 mM PMSF solution in ethanol was added and the entire supernatant was applied to centrifuge concentration columns (Viva Science, Lincoln, UK). Centrifugation was performed at 4000 rpm for 20 min yielding a final volume of 40 *µ*l. The identical volume of 2 x sample buffer was added and supernatant proteins were analyzed by SDS-PAGE.

### SDS-PAGE and Western blot analysis

SDS-PAGE and Western blot analysis were performed as described in Example 1.

### Reconstitution of the iron-sulfur cluster

Chemical reconstitution of the iron-sulfur cluster and refolding of Adx on the surface of *E. coli* cells was performed under strictly anaerobic conditions in 50 mM Tris-Cl buffer (pH 7.4) at ambient temperature as described in example 1. The bacterial suspension with a calculated final OD₅₇₈ of 50 (4 ml) was supplemented with 1 mM β-mercaptoethanol and 0.2 mM ferrous ammonium sulfate and was slowly titrated with 100 ml of a solution containing 100 mM Li₂S and 2 mM dithiothreitol (38). Cells were finally washed two times in 50 mM potassium phosphate buffer (pH 7.4) and used for whole cell activity assays.

### Protein purification

Recombinant AdR and CYP11A1 were purified as described (58). Protein concentration was calculated using ε₄₅₀=11.3 (mM cm)⁻¹ for AdR. The concentration of CYP11A1 was estimated by CO-difference spectra of reduced haemoproteins using ε₄₅₀ = 91 (mM cm)⁻¹. Bacteria for the expression of free Adx, not fused to the autotransporter domains, were grown as previously reported and recombinant adrenodoxin was purified as described (32,46).

### Whole cell activity assay

The Adx dependent cholesterol side chain cleavage activity of cytochrome CYP11A1 was assayed in an assembled system catalyzing the conversion of cholesterol to pregnenolone (68,67). Assays were performed for 30 min at 37°C in 50 mM potassium phosphate (pH 7.4) and contained 100 µl *E. coli* cells, 0.5 µM adrenodoxin reductase, 0.4 µM CYP11A1, 400 µM cholesterol, 60 µM NADPH and a NADPH regenerating system. After the side chain cleavage reaction, the steroids were converted into their corresponding 3-one-4-en forms by the addition of 2 Units/ml cholesterol oxidase and extracted with chloroform.

### HPLC

Extracted steroids were analyzed by reversed phase HPLC (Jasco LC800 System) using a 3.9 x 150 mm Waters Nova-Pak C₁₈ column with an isocratic solvent system of acetonitril/isopropanol (15: 1).

### Size exclusion chromatography

Size exclusion chromatography was performed on a FPLC system (Pharmacia Biotech) using a Superdex 75 HiLoad column with a flow rate of 45 cm/h. The buffer was 50 mM potassium phosphate pH 7.4 containing 150 mM NaCl. As calibration standards the followig proteins have been used: Aprotinin (6.5 kDa), Cytochrome C (12.4 kDa), Chymotrypsinogen A (25.0 kDa), Egg albumin (43.0 kDa) and BSA (68.0 kDa), respectively.

### 3.2 Results

### Fusion protein expression

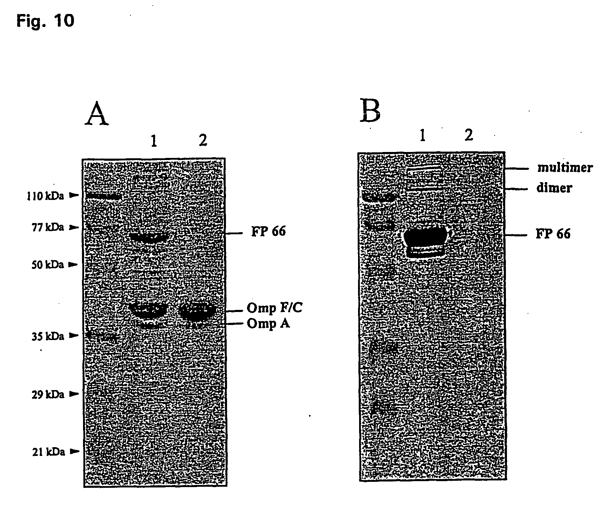

The efficient surface display of bovine adrenodoxin in *Escherichia coli* by the autotransporter pathway can be facilitated by the construction of an artificial gene. This gene encodes a fusion protein, consisting of Adx, a signal peptide and the translocation unit of the Adhesin involved in diffuse adhesion (AIDA-I), a natural autotransporter protein of *E. coli* (51) (Fig. 2B). The translocation unit contains the C terminal β-barrel and a linker region, that is necessary for full surface exposure of the passenger domain (15). To obtain Adx molecules anchored within the outer membrane by the transporting β-barrel, which results in surface display, the artificial gene had to be expressed in an outer membrane protease T (*ompT*) mutant of *E. coli.* OmpT cleaves proteins in the cell envelope of *E. coli* (42) and the linker region of AIDA-I contains a consensus cleavage site (RN) (15). In such cells, the full size fusion protein (FP66) could be easily detected by Coomassie staining of SDS polyacrylamide gels, when outer membrane preparations were applied (Fig. 10). Beside the facts that it was of the correct size and did not appear in the control without plasmid, the fusion protein could also be identified by labeling with an Adx specific antibody in Western blot experiments (Fig. 10B).

In addition to FP66 two protein bands with larger apparent molecular weight could be detected in Western blot (Fig. 10B lane 1). Although the determination of molecular weight for such large proteins tends to be not precise in our gels, they appeared to be in an adequate range for being multimers of the full size fusion protein. One protein band migrated quite close to the molecular weight of a dimer. Whether the second protein band could have been rather a trimer or a tetramer of FP66, can not be distinguished at this point. Both protein bands, however, were labeled by the Adx specific antibody, indicating that they contain the Adx passenger domain.

### Surface display and dimer formation of fusion proteins

To see whether these proteins were accessible at the surface of *E. coli,* trypsin was added to intact cells before outer membranes were prepared. As the outer membrane is not permeable for trypsin, degradation indicates surface exposure. As can be seen in Fig. 10 (lane 2, A and B) the full size Adx-containing fusion protein as well as the potential multimer bands disappeared. This seemed to be due to a complete degradation of the Adx passenger moiety, as bovine Adx is know to contain twenty trypsin consensus cleavage sites. The processing products, which are resistant to further trypsin digestion, could not be separated from OmpF/C within the polyacrylamide concentration applied. Therefore they only result in a more intensive coomassie staining of this band (Fig. 10A, lane 2).

In summary, beside the monomeric fusion protein FP66, we found two additional proteins in the outer membrane sample preparation of *E. coli* UT5600 pJJ004. They are in an adequate range for being multimers. Both contained the Adx domain and were processed by trypsin to the identical products as the monomeric FP66, indicating that they contain an identical translocation unit. As such additional protein bands did not appear when other passenger domains than Adx were applied with the same translocation unit (15), this was a hint, that we might have to deal with an Adx-driven dimerization or multimerization of a β-barrel containing fusion protein.

### Dimeric Adx on the surface

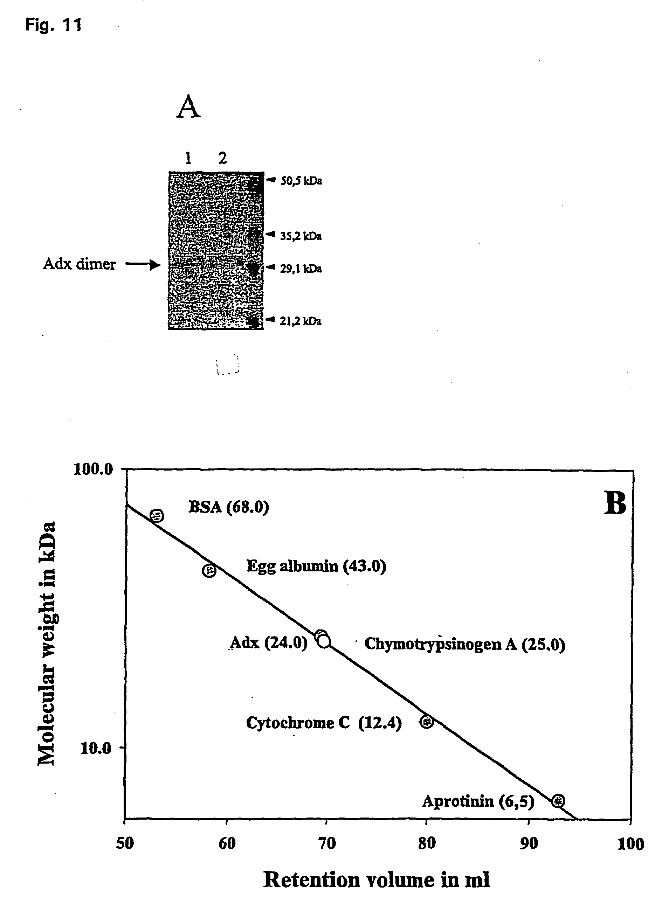

To verify this hypothesis, plasmid pJJ004, encoding FP66 was expressed in *E. coli* strain UT2300. This strain is isogenic to *E. coli* UT5600 with the exception that it is able to form an active outer membrane protease T (*omp*T⁺) (1). This results in the release of passenger domains, that are translocated to the surface by the autotransporter pathway into the supernatant (15) by cleavage within the linker region (Fig. 2B). In supernatants of *E. coli* UT2300 pJJ004 we could detect a protein that was labeled by the Adx specific antibody (Fig. 11). For this purpose cells were grown to the exponential growth phase, harvested, and resuspended into Tris/HCl buffer for 5 min. After centrifugation the supernatant was concentrated and subjected to SDS-PAGE and subsequent Western blotting. By this method a single protein band was detectable, that had an apparent molecular weight of around 32 kDa, corresponding in size to dimers of the Adx passenger protein. This indicated, that Adx is released from the translocation unit by OmpT as a dimer. The allover amount of free Adx molecules in the supernatant, however, appeared to be quite low in comparison to the large amount of fusion protein in *omp*T^{*+*} *E. coli* UT5600 (Fig. 10). Therefore outer membrane preparations from *omp*T^{*-*} UT2300 were also analysed for the fate of passenger Adx and full size fusion protein FP66. As can be seen in figure 12, processing of FP66 by OmpT was rather limited. The majority of fusion protein remained in its initial size within the outer membrane. This can explain, why we found only weak amounts of released passenger Adx in the supernatant. In addition, a protein was co-purified with the outer membrane that had an identical size with the supernatant Adx dimers and that was labeled by the Adx specific antibody. This means, that on one hand OmpT has only weakly processed FP66 and on the other, that the majority of released Adx passenger domains remained for some reasons associated with the outer membrane. To see whether both proteins were indeed exposed to the surface, trypsin was added to UT2300 pJJ004 cells before outer membranes were prepared. This resulted in the degradation of both proteins indicating their surface accessibility (Fig. 12 and 12B, lane 2).

In order to find out, whether OmpT processing of FP66 and the release of passenger Adx could be improved, growth conditions were varied for *E. coli* UT2300 pJJ004. In figure 13 outer membrane preparations from cells grown under different conditions are shown. Expression of FP66 was best, when cells were grown in the presence of 2-mercaptoethanol, glucose and trace elements. OmpT activity, however, was not substantially altered. The ratio of processed to unprocessed FP66 remained nearly constant. Possibly due to the better expression of FP66 in cells of lane 4, also monomers of Adx were detectable. In lane 3, a faint band indicated the monomer too, but it was almost below the detection limit. In summary, the attempt to improve the supernatant content of passenger Adx by altering the growth conditions failed. OmpT activity was not stimulated by this strategy, and as a consequence, the number of Adx molecules in the supernatant did not substantially increase (not shown). It cannot be excluded, that some monomeric Adx molecules might have been in the supernatant too. Supposed that the ratio dimer/monomer in the supernatant is identical as seen in figure 13 (lane 4), the monomers were most probably below the detection limit.

In order to find out, whether free Adx, that is not connected to the autotransporter domains, can also form dimers, bovine Adx was purified after recombinant expression in *E. coli* and subjected to size exclusion chromatography. As can be seen in Fig. 11B, the apparent molecular weight of free Adx determined by this method (24 kDa) was quite close to the calculated molecular weight of a dimeric molecule (28,8 kDa) indicating, that indeed free Adx also forms dimers.

Reconstitution of the iron-sulfur cluster has no influence on Adx dimer formation on the bacterial surface In Example 1 we showed that the iron sulfur-cluster can be effectively incorporated into apo-Adx displayed on the *E. coli* coli surface. Adx devoid of the 2[Fe-S] cluster is biologically not active. Due to the autotransporter secretion mechanism, however, Adx can only be transported to the surface in an unfolded state as apo-Adx without prosthetic group. Iron-sulfur cluster incorporation appeared to be best under anaerobic conditions, when LiS₂ was added dropwise to Adx-expressing cells in a ferrous ammonium sulfate buffer at room temperature. By this procedure iron sulfur clusters were formed and immediately incorporated into apo-Adx displayed at the bacterial surface. Cells survived this procedure and could be handled afterwards under aerobic conditions without loss of activity. *E. coli* UT5600 pJJ004 as well as *E. coli* UT2300 pJJ004 were treated this way and outer membranes were prepared and analyzed by SDS-PAGE and Western blotting. The results were identical to those obtained before chemical reconstitution (Fig. 10, Fig. 12). In *E. coli* UT5600 pJJ004, multimers of the full size fusion protein were detectable, and in *E. coli* UT2300 pJJ004 the majority of Adx, released from the transporter unit, remained associated to the surface as dimers (not shown). This indicates, that dimer formation is not a special property of apo-Adx, devoid of the iron-sulfur cluster and that iron-sulfur cluster incorporation does not affect dimer formation.

### Whole cell steroid synthesis

To whole cells of *E. coli* UT5600 pJJ004, expressing dimeric holo-Adx, purified adrenodoxin reductase (AdR) and CYP11A1, were added without detergents to find out what minimal number of components would allow steroid conversion. CYP11A1, the side chain cleaving enzyme, converts cholesterol to pregnenolone (69). Therefore cholesterol was supplied as substrate and, to maintain sufficient reduction equivalents throughout the entire reaction, a NADPH-regenerating system based on glucose-6-phoshate dehydrogenase was additionally provided. HPLC analysis of the reaction revealed significant pregnenolone formation. By integration of the product peak, the activity was calculated to be 0.21 nm/h/nmol CYP11A1, which is in the same order of magnitude as it has been determined for detergent based steroid conversion assays. This indicates, that whole cells expressing functional Adx on the surface, supply a sufficient environment for a P450 enzyme (CYP11A1) to be active. Addition of detergents as before (68) or even constitution of membrane vesicles (63) seems to be not necessary. As contact between AdR, Adx and CYP11A1 is required to obtain electron transfer (40), Adx molecules displayed may direct its reaction partners to the bacterial surface, yielding an easy to handle whole-cell steroid synthesis nanofactory (Fig. 14). This principle approach applied here for CYP11A1 could be transfered to many other P450 enzymes in order to synthezise a wide variety of organic molecules (Table 1).

### 3.3. Discussion

From the present investigation, two important observations can be derived. First it shows, that a recombinant passenger protein, bovine adrenodoxin (Adx) translocated by the AIDA-I autotransporter unit, can form dimers on the surface. More recently crystal structure analysis revealed bovine Adx to be a dimer (50). Functional considerations on the electron transfer from one enzyme (AdR) to another (P450) via Adx, implicated that one Adx molecule is in direct contact with AdR and a second with P450, having an Adx-Adx interface in between. Therefore it seems plausible, that the natural affinity of Adx passenger monomers directs the β-barrel containing fusion proteins FP66 to contact. As the number of Adx molecules displayed on the surface has been determined to be more than 10⁵ (see Example 1), they can be assumed to obtain sufficient vicinity. Because they should be freely motile, the β-barrels within the outer membrane cannot offer much resistance against this affinity of passengers. To our knowledge, this is the first report on the functional, passenger driven, dimerization of a protein on the surface of *E. coli.*

It is worth emphasizing that Adx molecules are initially expressed as monomers, from monomeric genes. Dimerization is self-directed and does not require any connection in between of Adx monomers by a linker peptide, as applied for so-called single chain antibodies (55). Therefore the autotransporter mediated surface display of single polypeptides that can dimerize or multimerize on the surface offer new dimensions in the field of biotechnology applications as e.g. antibody technology.

At this point, it cannot be excluded that there might also be a β-barrel driven multimerization of FP66. The autotransporter β-barrel is structurally related to the β-barrel of the so-called porins, channels for small hydrophilic molecules within the outer membrane. The porins, like OmpF, have been shown to form trimers and monomers are assumed to be thermodynamically unstable (65). As we never observed a multimerization of fusion proteins or released passenger domains that withstood SDS-PAGE, when another passenger protein then Adx was expressed, the stable interaction in our experiments must be due to Adx itself. A mixed scenario, in which e.g. the autotransporter β-barrel propagates approximation of the passenger domains to finally form stable dimers by self-contact, might be conceivable. But up to now there is no experimental evidence.

Bovine Adx contains five cysteines of which four are involved in the iron-sulfur cluster binding. Theoretically the fifth cysteine could be available for disulfide bonding. From the crystal data (50), however, this can be excluded. The autotransporter used in this Adx-autotransporter fusion contained no cysteines at all (15). Therefore in our experiments, it is very unlikely, that dimerization results from disulfide bonding. This means, that dimer formation is due to an interaction or bonding that is stable enough to withstand 20 min of boiling and is resolved by the addition of β-mercaptoethanol but is no disulfide bond. An obvious explanation could be, that β-mercaptoethanol is reacting with the fifth cysteine, causing a conformational change, that weakens the strong interaction within the two partners of the dimer. In any way, this could indicate, that in general there might be strong interactions between protein domains, that can be resolved by treatment with β-mercaptoethanol, but are no disulfide bonds.

Stable protein dimers under conditions of SDS gelelectrophoresis with reducing agents have been described for glycophorin A (66) and γ-glutamyltranspeptidase (59). In glycophorin A a methionine residue plays the important role for dimerization by hydrophobic interactions. For *γ-*glutamyltranspeptidase strong ionic and/or hydrophobic interactions were discussed, whereas in both cases disulfide bonds seem not to be involved.

By size exclusion chromatography, we could verify, that also free Adx is able to form dimers. This indicates, that dimerization is not a special feature of Adx-autotransporter fusion proteins, but seems to be relevant for the function of Adx in general.

The second important observation of our investigation is, that whole cells expressing functional Adx molecules on the surface provide sufficient environment for P450 enzymes, that are naturally membrane-associated, to function. The addition of detergents or the reconstitution of membrane vesicles, is not necessary. This offers substantial improvements in accessing the biotechnological potential of P450 enzymes. *E. coli* cells expressing Adx at the surface could be supplied with P450 and AdR and then serve as a whole cell carrier to target e.g. soil that needs detoxification. Cells that are prepared likewise can be used in a fermenter for the synthesis of steroids or plant or microorganism secondary metabolites. Laboratory evolution could also be simplified, as cells can easily be harvested by centrifugation and - if needed - distributed in separate reaction chambers.

### References

1. Reetz, M.T. Evolution in the test tube as a means to create enantioselective enzymes for use in organic synthesis. *Sci Prog* **83**, 157-172 (2000).
2. Schreuder, M.P., Mooren, A.T., Toschka, H.Y., Verrips, C.T. & Klis, F.M. Immobilizing proteins on the surface of yeast cells. *Trends Biotechnol* **14,** 115-120 (1996).
3. Murai, T. et al. Development of an arming yeast strain for efficient utilization of starch by co-display of sequential amylolytic enzymes on the cell surface. *Appl Microbiol Biotechnol* **51,** 65-70 (1999).
4. Strauss, A. & Gotz, F. In vivo immobilization of enzymatically active polypeptides on the cell surface of *Staphylococcus carnosus. Mol Microbiol* **21**, 491-500 (1996).
5. Stahl, S. & Uhlen, M. Bacterial surface display: trends and progress. *Trends Biotechnol* **15,** 185-192 (1997).
6. Georgiou, G. et al. Display of heterologous proteins on the surface of microorganisms: from the screening of combinatorial libraries to live recombinant vaccines. *Nat Biotechnol* **15,** 29-34 (1997).
7. Westerlund-Wikstrom, B. Peptide display on bacterial flagella: principles and applications. *Int J Med Microbiol* **290,** 223-230 (2000).
8. Valls, M., Atrian, S., de Lorenzo, V. & Fernandez, L.A. Engineering a mouse metallothionein on the cell surface of ralstonia eutropha CH34 for immobilization of heavy metals in soil. *Nat Biotechnol* **18**, 661-665 (2000).
9. Stathopoulos, C., Georgiou, G. & Earhart, C.F. Characterization of *Escherichia coli* expressing an Lpp'OmpA(46-159)-PhoA fusion protein localized in the outer membrane. *Appl Microbiol Biotechnol* **45**, 112-119 (1996).
10. Lu, Z., Tripp, B.C. & McCoy, J.M. Displaying libraries of conformationally constrained peptides on the surface of *Escherichia coli* as flagellin fusions. *Methods. Mol Biol* **87**, 265-280 (1998).
11. Klemm, P. & Schembri, M.A. Fimbriae-assisted bacterial surface display of heterologous peptides. *Int J Med Microbiol* **290,** 215-221 (2000).
12. Jung, H.C., Lebeault, J.M. & Pan, J.G. Surface display of *Zymomonas mobilis* levansucrase by using the ice-nucleation protein of *Pseudomonas syringae. Nat Biotechnol* **16**, 576-580 (1998).
13. Georgiou, G. et al. Display of beta-lactamase on the *Escherichia coli* surface: outer membrane phenotypes conferred by Lpp'-OmpA'-beta-lactamase fusions. *Protein Eng* **9,** 239-247 (1996).
14. Francisco, J.A., Earhart, C.F. & Georgiou, G. Transport and anchoring of beta-lactamase to the external surface of *Escherichia coli. Proc Natl Acad Sci U S A* **89,** 2713-2717 (1992).
15. Maurer, J., Jose, J. & Meyer, T.F. Autodisplay: one-component system for efficient surface display and release of soluble recombinant proteins from *Escherichia coli. J Bacteriol* **179,** 794-804 (1997).
16. Lattemann, C.T., Maurer, J., Gerland, E. & Meyer, T.F. Autodisplay: functional display of active beta-lactamase on the surface of *Escherichia coli* by the AIDA-I autotransporter. *J Bacteriol **182,*** 3726-3733 (2000).
17. Jose, J., Jahnig, F. & Meyer, T.F. Common structural features of IgA1 protease-like outer membrane protein autotransporters. *Mol Microbiol* **18,** 378-380 (1995).
18. Henderson, I.R., Navarro-Garcia, F. & Nataro, J.P. The great escape: structure and function of the autotransporter proteins. *Trends Microbiol* **6**, 370-378 (1998).
19. Loveless, B.J. & Saier, M.H., Jr. A novel family of channel-forming, autotransporting, bacterial virulence factors. *Mol Membr Biol* **14***,* 113-123 (1997).
20. Pohlner, J., Halter, R., Beyreuther, K. & Meyer, T.F. Gene structure and extracellular secretion of *Neisseria gonorrhoeae* IgA protease. *Nature* **325**, 458-462 (1987).
21. Maurer, J., Jose, J. & Meyer, T.F. Characterization of the essential transport function of the AIDA-I autotransporter and evidence supporting structural predictions. *J Bacteriol* **181**, 7014-7020 (1999).
22. Shannon, J.L. & Fernandez, R.C. The C-terminal domain of the *Bordetella pertussis* autotransporter BrkA forms a pore in lipid bilayer membranes. *J Bacteriol* **181,** 5838-5842 (1999).
23. Klauser, T., Pohlner, J. & Meyer, T.F. Selective extracellular release of cholera toxin B subunit by *Escherichia coli*: dissection of Neisseria Iga beta-mediated outer membrane transport. *Embo J* **11,** 2327-2335 (1992).
24. Klauser, T., Pohlner, J. & Meyer, T.F. Extracellular transport of cholera toxin B subunit using Neisseria IgA protease beta-domain: conformation-dependent outer membrane translocation. *Embo J* **9**, 1991-1999 (1990).
25. Shimada, K., Ohnishi, Y., Horinouchi, S. & Beppu, T. Extracellular transport of pseudoazurin of *Alcaligenes faecalis* in *Escherichia coli* using the COOH-terminal domain of Serratia marcescens serine protease. *J Biochem* **116,** 327-334 (1994).
26. Wentzel, A., Christmann, A., Kratzner, R. & Kolmar, H. Sequence requirements of the GPNG beta-turn of the *Ecballium elaterium* trypsin inhibitor II explored by combinatorial library screening. *J Biol Chem* **274**, 21037-21043 (1999).
27. Veiga, E., de Lorenzo, V. & Fernandez, L.A. Probing secretion and translocation of a beta-autotransporter using a reporter single-chain Fv as a cognate passenger domain. *Mol Microbiol* **33**, 1232-1243 (1999).
28. Konieczny, M.P., Suhr, M., Noll, A., Autenrieth, I. B. & Alexander Schmidt, M. Cell surface presentation of recombinant (poly-) peptides including functional T-cell epitopes by the AIDA autotransporter system. *FEMS Immunol Med Microbiol* **27**, 321-332 (2000).
29. Grinberg, A.V., Hannemann, F., Schiffler, B., Müller, J., Heinemann, U., and Bernhardt, R. Adrenodoxin: structure, stability, and electron transfer properties. *Proteins* **40**, 590-612 (2000).
30. Estabrook, R.W. et al. in *Iron-Sulfur Proteins,* Vol. 2 193-223 (Academic Press, New York; 1973).
31. Muller, A. et al. New aspects of electron transfer revealed by the crystal structure of a truncated bovine adrenodoxin, Adx(4-108). *Structure* **6**, 269-280 (1998).
32. Uhlmann, H., Beckert, V., Schwarz, D. & Bernhardt, R. Expression of bovine adrenodoxin in *E. coli* and site-directed mutagenesis of /2 Fe-2S/ cluster ligands. *Biochem Biophys Res Commun* **188,** 1131-1138 (1992).
33. Baneyx, F. & Georgiou, G. Degradation of secreted proteins in *Escherichia coli. Ann N Y Acad Sci* **665,** 301-308 (1992).
34. Jose, J., Kramer, J., Klauser, T., Pohlner, J. & Meyer, T.F. Absence of periplasmic DsbA oxidoreductase facilitates export of cysteine-containing passenger proteins to the *Escherichia coli* cell surface via the Iga beta autotransporter pathway. *Gene* **178,** 107-110 (1996).
35. Goder, V., Beckert, V., Pfeil, W. & Bernhardt, R. Impact of the presequence of a mitochondrium-targeted precursor, preadrenodoxin, on folding, catalytic activity, and stability of the protein in vitro. *Arch Biochem Biophys* **359**, 31-41 (1998).
36. Matsubara, H. & Saeki, K. Sructural and functional diversity of ferredoxins and related proteins. *Adv Inorg Chem* **38**, 223-280 (1992).
37. lametti S., Uhlmann H., Sala N., Bernhardt, R., Ragg E., Bonomi F., Reversible, non-denaturing metal substitution in bovine adrenodoxin and spinach ferredoxin and the different reactivities of [2Fe-2S]-cluster-containing proteins. *Eur J Biochem* **239** (3):818-26.
38. Bonomi, F., Pagani, S. & Kurtz, D.M., Jr. Enzymic synthesis of the 4Fe-4S clusters of *Clostridium pasteurianum* ferredoxin. *Eur J Biochem* **148**, 67-73 (1985).
39. Koebnik, R., Locher, K.P. & Van Gelder, P. Structure and function of bacterial outer membrane proteins: barrels in a nutshell. *Mol Microbiol* **37**, 239-253 (2000).
40. Vickery, L.E. Molecular recognition and electron transfer in mitochondrial steroid hydroxylase systems. *Steroids* **62**, 124-127 (1997).
41. Bernhardt, R. Cytochrome P450: structure, function, and generation of reactive oxygen species. *Rev Physiol Biochem Pharmacol* **127**, 137-221 (1996).
42. Grodberg, J. & Dunn, J.J. ompT encodes the *Escherichia coli* outer membrane protease that cleaves T7 RNA polymerase during purification. *J Bacteriol* **170,** 1245-1253 (1988).
43. Uhlmann, H., Beckert, V., Schwarz, D. & Bernhardt, R. Expression of bovine adrenodoxin in *E. coli* and site-directed mutagenesis of /2 Fe-2S/ cluster ligands. *Biochem Biophys Res Commun* **188,** 1131-1138 (1992).
44. Hantke, K. Regulation of ferric iron transport in *Escherichia coli* K12: isolation of a constitutive mutant. *Mol Gen Genet* **182,** 288-292 (1981).
45. Bonomi, F., Werth, M. T., and Kurtz, D. M. Jr Assembly of FenSn(SR)4-2 (n = 2,4) in aqueous media from iron salts, thiols, and sulfur, sulfide or thiosulfate plus rhodanese. *Inor Chem* **24,** 4431-4435 (1985).
46. Sagara, Y. et al. Direct expression of adrenodoxin reductase in *Escherichia coli* and the functional characterization. *Biol Pharm Bull* **16**, 627-630 (1993).
47. Kimura, T. & Suzuki, K. Components of the electron transport system in adrenal steroid hydroxylase. Isolation and properties of non-heme iron protein (adrenodoxin). *J Biol Chem* **242,** 485-491 (1967).
48. Hiwatashi, A., Ichikawa, Y., Yamano, T. & Maruya, N. Properties of crystalline reduced nicotinamide adenine dinucleotide phosphate-adrenodoxin reductase from bovine adrenocortical mitochondria. II. Essential histidyl and cysteinyl residues at the NADPH binding site of NADPH-adrenodoxin reductase. *Biochemistry* **15**, 3091-3097 (1976).
49. Akhrem, A.A., Lapko, V.N., Lapko, A.G., Shkumatov, V.M. & Chashchin, V.L. Isolation, structural organization and mechanism of action of mitochondrial steroid hydroxylating systems. *Acta Biol Med Ger* **38**, 257-273 (1979).
50. Pikuleva, I.A., Tesh, K., Waterman, M.R. & Kim, Y. The tertiary structure of full-length bovine adrenodoxin suggests functional dimers. *Arch.Biochem.Biophys.* **373**, 44-55 (2000).
51. Benz, I., Schmidt, M.A. (1992) Isolation and serologic characterization of AIDA-1, the adhesin mediating the diffuse adherence phenotype of the diarrhea-associated *Escherichia coli* strain 2787 (0126:H27). Infect. Immun. 60:13-18
52. Cali, J.J., Russell, D.W. (1991) Characterization of human sterol 27-hydroxylase. A mitochondrial cytochrome P-450 that catalyzes multiple oxidation reaction in bile acid biosynthesis. J. Biol. Chem. 266:7774-7778.
53. Chen, K.S., Prahl J.M., DeLuca, H.F. (1993) Isolation and expression of human 1,25-dihydroxyvitamin D3 24-hydroxylase cDNA. Proc. Natl. Acad. Sci. U S A 90:4543-4547.
54. Cherry, J.R. (2000) Directed evolution of microbial oxidative enzymes. Curr. Opin. Biotechnol. 11:250-254.
55. Daugherty, P.S., Chen, G., Iverson, B.L., Georgiou, G. (2000) Quantitative analysis of the effect of the mutation frequency on the affinity maturation of single chain Fv antibodies. Proc. Natl. Acad. Sci. U S A 97:2029-2034
56. Guo, Y.D., Strugnell, S., Back, D.W., Jones, G. (1993) Transfected human liver cytochrome P-450 hydroxylates vitamin D analogs at different side-chain positions. Proc. Natl. Acad. Sci. U S A 90:8668-8672.
57. Guzov, V.M., Unnithan, G.C., Chernogolov, A.A., Feyereisen, R. (1998) CYP12A1, a mitochondrial cytochrome P450 from the house fly. Arch. Biochem. Biophys. 359:231-240.
58. Hannemann, F., Rottmann, M., Schiffler, B., Zapp, J., Bernhardt, R. (2001) The Loop Region Covering the Iron-Sulfur Cluster in Bovine Adrenodoxin Comprises a New Interaction Site for Redox Partners. J. Biol. Chem. 276:1369-1375
59. Hughey, R.P., Altman, R.A., Wells, W.J., Curto, K.A. (1986) Evidence for stable homodimers and heterodimers of gamma-glutamyltranspeptidase subunits under protein-denaturing conditions. Biochim. Biophys. Acta 874:150-159.
60. Nelson, D.R. et al. (1996) P450 superfamily: update on new sequences, gene mapping, accession numbers and nomenclature. Pharmacogenetics 6:1-42.
61. Okamoto, M., Nonaka, Y. (1990) Structure and function of adrenal mitochondrial Cytochrome P-4501β. In: Ruckpaul K, Rein H (eds). Akademie Verlag, Berlin, Germany, pp 127-152
62. Okamoto, M., Nonaka, Y. (1992) Molecular biology of rat steroid 11 beta-hydroxylase [P450(11 beta)] and aldosterone synthase [P450(11 beta, aldo)]. J. Steroid Biochem. Mol. Biol. 41:415-419.
63. Roberts, G.C. (1999) The power of evolution: accessing the synthetic potential of P450s. Chem. Biol. 6:R269-272.
64. Robin, M.A., An and atheerthava rada, H.K., Fang, J.K., Cudic, M., Otvos, L., Avadhani, N.G. (2001) Mitochondrial targeted cytochrome P450 2E1 (P450MT5) contains an intact N terminus and requires mitochondrial specific electron transfer proteins for activity. J. Biol. Chem. 276:24680-24689.
65. Schulz, G.E. (2000) Beta-Barrel membrane proteins. Curr. Opin. Struct. Biol. 10:443447.
66. Silverberg, M., Furthmayr, H., Marchesi, V.T. (1976) The effect of carboxymethylating a single methionine residue on the subunit interactions of glycophorin A. Biochemistry 15:1448-1454.
67. Sugano, S., Miura, R., Morishima, N. (1996) Identification of intermediates in the conversion of cholesterol to pregnenolone with a reconstituted cytochrome p450scc system: accumulation of the intermediate modulated by the adrenodoxin level. J. Biochem. 120:780-787
68. Uhlmann, H., Kraft, R., Bernhardt, R. (1994) C-terminal region of adrenodoxin affects its structural integrity and determines differences in its electron transfer function to cytochrome P-450. J. Biol. Chem. 269:22557-22564
69. Usanov, S.A., Chashchin, V.L., Akhrem, A.A. (1990) Cytochrome P-450 dependent pathways of the biosynthesis of steroid hormones. In: Ruckpaul K, Rein H (eds). Akademie Verlag, Berlin, Germany, pp 1-57

**Table 1. Active Adx molecules on the surface of viable E. coli cells after reconstitution at different temperatures**

| *reconstitution temperature* | *cells applied*/*ml* | *living cells*/*ml after reco* | *nmol Adx*/*ml* | *nmol Adx*/*living cell* | *Adx molecules*/*living cell* | *Adx molecules*/*cells applied* |
|---|---|---|---|---|---|---|
| - | | | | | | |
| 22° C | 1,4 x 10¹⁰ | 2,0 x 10¹⁰ | 0 | 0 | 0 | 0 |
| | | | | | | |
| + | | | | | | |
| 22° C | 1,3 x 10¹⁰ | 1,1 x 10¹⁰ | 3.25 | 2.95 x 10⁻¹⁰ | 1.8 x 10⁵ | 1.5 x 10⁵ |
| | | | | | | |
| + | | | | | | |
| 40° C | 1,5 x 10¹⁰ | 2,1 x 10¹⁰ | 2.75 | 1.31 x 10⁻¹⁰ | 0.8 x 10⁵ | 1.1 x 10⁵ |
| | | | | | | |
| + | | | | | | |
| 60° C | 1,1 x 10¹⁰ | 1,5 x 10¹⁰ | 1.75 | 1.16 x 10⁻¹⁰ | 0.7 x 10⁵ | 0.9 x 10⁵ |
| | | | | | | |
| + | | | | | | |
| 70° C | 1,3 x 10¹⁰ | 0,7 x 10⁷ | 0.75 | 1.10 x 10⁻⁸ | 6.6 x 10⁷ | 0.3 x 10⁵ |

### Table 2

**Table 1. Substrates of Adx dependent cytochrome P-450 catalyzed reactions**

| | |
|---|---|
| **CYP2E1**^{**a**} | metabolizes a wide variety of chemicals with different structures including both endogenous substrates, such as ethanol, acetone, and acetal, as well as exogenous substrates including benzene, carbon tetrachloride, ethylene glycol, and premutagens like nitrosamines (Robin, et al. 2001). |
| | |
| **CYP11A1** | catalyzes the first and rate limiting step in steroid hormones biosynthesis, the cholesterol side chain cleavage of cholestrol to form pregnenolone; wide substrate specificity with respect to the length of the side chain and the position of the hydroxygroup (Usanov, et al. 1990). |
| | |
| **CYP11B1** | involved in biosynthesis of the main corticosteroid cortisol, catalyzes the 11β-hydroxylation of 11-deoxycortisol to form cortisol (Okamoto and Nonaka 1990). |
| | |
| **CYP11B2** | involved in biosynthesis of the main minaralcorticoid aldosterone, catalyzes the 11β-hydroxylation of deoxycorticosterone to cortisone and the following 18-oxidation to form aldosterone (Okamoto and Nonaka 1992). |
| | |
| **CYP12A1** | involved in steroid metabolism of insect cells; metabolizes a number of insecticides and several xenobiotics; catalyzes different types of chemical reactions such as hydroxylations, epoxidation, N-demethylation, O-alkylation, desulfuration, and oxidative ester cleavage (Guzov, et al. 1998). |
| | |
| **CYP24** | initiates the degradation of 1,25-dihydroxyvitamin D3, the physiologically active form of vitamin D3, by hydroxylation of the side chain (Chen, et al. 1993). |
| | |
| **CYP27** | involved in the bile acid biosynthetic pathways catalyzing 27-hydroxylation and multiple oxidation reactions at the C-27 atom of steroids and is involved in the activation of vitamin D, catalyzes 24-, 25-, and 26(27)-hydroxylation reactions in Vitamine D derivatives (Cali and Russell 1991) (Guo, et al. 1993). |

| | |
|---|---|
| ^{a}) Systematic abbreviation according to (Nelson, et al. 1996) | |

## Claims

1. A method for displaying a recombinant polypeptide containing a prosthetic group on the surface of a host cell comprising the steps:
(a) providing a host cell transformed with a nucleic acid fusion operatively linked with an expression control sequence said nucleic acid fusion comprising:
(i) a portion encoding a signal peptide,
(ii) a portion encoding the recombinant polypeptide to be displayed,
(iii) optionally a portion encoding a protease recognition site,
(iv) a portion encoding a transmembrane linker, and
(v) a portion encoding the transporter domain of an autotransporter,
(b) culturing the host cell under conditions wherein the nucleic acid fusion is expressed and the expression product comprising the recombinant polypeptide is displayed on the surface of the host cell, and
(c) contacting the recombinant polypeptide with a prosthetic group under conditions wherein the prosthetic group combines with the recombinant polypeptide and a functional recombinant polypeptide containing the prosthetic group is formed.

2. The method according to claim 1 wherein the prosthetic group comprises an inorganic component.

3. The method according to claim 2 wherein the prosthetic group is a metal containing group.

4. The method according to claim 3 wherein the metal is selected from cobalt, nickel, manganese, copper and iron.

5. The method according to claim 4 wherein the prosthetic group is selected from [2Fe-2S] clusters, [4Fe-4S] clusters and metal porphyrin, e.g. heme groups.

6. The method according to claim 1 wherein the prostethic group comprises an organic component.

7. The method according to claim 6 wherein the prosthetic group is selected from flavin containing groups, e.g. FMN or FAD, nicotin containing groups, e.g. NAD, NADH, NADP or NADPH, biotin, α2-microglobulin, thiamine pyrophosphate, coenzyme A, pyridoxal phosphate, coenzyme B12, biocytine, tetrahydrofolate and lipoic acid.

8. The method according to any one of claims 1-7 wherein the prosthetic group is combined with the recombinant polypeptide on the surface of the host cell.

9. The method according to any one of claims 1-7 wherein the prosthetic group is combined with the recombinant polypeptide on a membrane preparation derived from the host cell.

10. The method according to any one of claims 1-7 wherein the prosthetic group is combined with the recombinant polypeptide after cleavage of the recombinant polypeptide from the host cell or a membrane preparation thereof.

11. A method for displaying a recombinant multimeric polypeptide on the surface of a host cell comprising the steps:
(a) providing a host cell transformed with a nucleic acid fusion operatively linked with an expression control sequence said nucleic acid fusion comprising:
(i) a portion encoding a signal peptide,
(ii) a portion encoding a subunit of the multimeric polypeptide to be displayed,
(iii) optionally a portion encoding a protease recognition site,
(iv) a portion encoding a transmembrane linker, and
(v) a portion encoding the transporter domain of an autotransporter,
(b) culturing the host cell under conditions wherein the nucleic acid fusion is expressed and the expression product comprising the subunit of the multimeric recombinant polypeptide is displayed on the surface of the host cell, and
(c) combining the displayed subunit with at least one further subunit of the multimeric recombinant polypeptide and forming a functional multimeric recombinant polypeptide on the surface of the host cell.

12. The method according to claim 11 wherein the multimeric recombinant polypeptide is a homodimer or a homomultimer.

13. The method according to claim 11 wherein the multimeric recombinant polypeptide is a heterodimer or a heteromultimer.

14. The method according to any one of claims 11-13 wherein at least one subunit of the multimeric recombinant protein contains a prosthetic group.

15. The method according to any one of claims 11-14 wherein a homodimer or a homomultimer is formed by an association of several polypeptide subunits displayed on the host cell membrane.

16. The method according to any one of claims 11-15 wherein a heterodimer or a heteromultimer is formed by an association of several different polypeptide subunits displayed on the host cell membrane.

17. The method according to any one of claims 11-14 wherein a multimeric recombinant polypeptide is formed by an association of at least one polypeptide subunit displayed on the host cell membrane and at least one soluble polypeptide subunit added to the host cell membrane.

18. The method according to claim 17 wherein the added subunit is different from the displayed subunit.

19. The method according to any one of the previous claims wherein the host cell is a bacterium.

20. The method according to claim 19 wherein the bacterium is a gram-negative bacterium, particularly an enterobacterium, e.g. *E. coli.*

21. The method according to any one of the previous claims wherein the transporter domain of the autotransporter forms a β-barrel structure.

22. The method according to claim 21 wherein the transporter domain of the autotransporter is selected from the *E. coli* AIDA-I protein, the *Shigella flexneri* Sep A protein, the *Shigella flexneri* IcsA protein, the *E. coli* Tsh protein, the *Serratia marcescens* Ssp protein, the *Helicobacter mustelae* Hsr protein, the *Bordetella ssp.* Prn protein, the *Haemophilus influenzae* Hap protein, the *Bordetalla pertussis* Brk A protein, the *Helicobacter pylori Vac* A protein, the surface protein SpaP, rOmpB or SipT from *Rickettsia,* the IgA protease from *Neisseria* or *Haemophilus* and variants thereof.

23. The method according to claim 22 wherein the transporter domain of the autotransporter is the *E. coli* AIDA-I protein or a variant thereof.

24. Host cell displaying a functional recombinant polypeptide on the surface thereof wherein the recombinant polypeptide contains a prosthetic group.

25. The host cell of claim 24 wherein the recombinant polypeptide is displayed by the transporter domain of an autotransporter.

26. The host cell of claim 24 or 25 wherein the polypeptide is selected from ferredoxins, P450 reductases, cytochrome b5, P450 enzymes, flavoproteins and any combinations thereof.

27. Host cell displaying a functional recombinant polypeptide on the surface thereof wherein the recombinant polypeptide is a multimeric polypeptide containing at least two polypeptide subunits.

28. The host cell of claim 27 wherein at least one subunit of the multimeric polypeptide is displayed by the transporter domain of an autotransporter.

29. The host cell of any one of claims 24-28 which is a bacterial host cell, particularly a gram-negative bacterial host cell.

30. Membrane preparation which is derived from a host cell of any one of claims 24-29.

31. Use of a cell of any one of claims 24-29 or a membrane preparation of claim 30 for a chemical synthesis procedure.

32. Use of claim 31 for the synthesis of organic substances selected from enzyme substrates, drugs, hormones, starting materials and intermediates for synthesis procedures and chiral substances.

33. Use of a cell of any one of claims 24-29 or a membrane preparation of claim 30 for a directed evolution procedure.

34. Use of a cell of any one of claims 24-29 or a membrane preparation of claim 30 as an assay system for a screening procedure, e.g. for identifying modulators of P450 enzymes.

35. Use of a cell of any one of claims 24-29 or a membrane preparation of claim 30 as a system for toxicity monitoring.

36. Use of a cell of any one of claims 24-29 or a membrane preparation of claim 30 as a system for degrading toxic substances.

## Patentansprüche

1. Verfahren zur Exposition eines rekombinanten Polypeptids enthaltend eine prosthetische Gruppe auf der Oberfläche einer Wirtszelle umfassend die Schritte:
(a) Bereitstellen einer Wirtszelle transformiert mit einer Nukleinsäurefusion operativ verbunden mit einer Expressionskontrollsequenz, wobei die Nukleinsäurefusion umfasst:
(i) einen Anteil kodierend ein Signalpeptid,
(ii) einen Anteil kodierend das rekombinante Polypeptid, welches exponiert werden soll,
(iii) gegebenenfalls einen Anteil kodierend eine Proteaseerkennungsstelle,
(iv) einen Anteil kodierend einen Transmembranlinker, und
(v) einen Anteil kodierend die Transporterdomäne eines Autotransporters,
(b) Kultivieren der Wirtszelle unter Bedingungen, unter denen die Nukleinsäurefusion exprimiert wird und das Expressionsprodukt umfassend das rekombinante Polypeptid auf der Oberfläche der Wirtszelle exponiert wird, und
(c) Inkontaktbringen des rekombinanten Polypeptids mit einer prosthetischen Gruppe unter Bedingungen, unter denen die prosthetische Gruppe mit dem rekombinanten Polypeptid verbunden wird und ein funktionelles rekombinantes Polypeptid enthaltend die prosthetische Gruppe gebildet wird.

2. Verfahren gemäß Anspruch 1, wobei die prosthetische Gruppe einen anorganischen Bestandteil umfasst.

3. Verfahren gemäß Anspruch 2, wobei die prosthetische Gruppe eine metallenthaltende Gruppe ist.

4. Verfahren gemäß Anspruch 3, wobei das Metall aus Kobalt, Nickel, Mangan, Kupfer und Eisen ausgewählt wird.

5. Verfahren gemäß Anspruch 4, wobei die prosthetische Gruppe ausgewählt wird aus [2Fe-2S]-Clustern, [4Fe-4S]-Clustern und Metallporphyrin, z. B. Hämgruppen.

6. Verfahren gemäß Anspruch 1, wobei die prosthetische Gruppe einen organischen Bestandteil umfasst.

7. Verfahren gemäß Anspruch 6, wobei die prosthetische Gruppe ausgewählt wird aus flavinenthaltenden Gruppen, z. B. FMN oder FAD, nicotinenthaltenden Gruppen, z. B. NAD, NADH, NADP oder NADPH, Biotin, α2-Microglobulin, Thiaminpyrophosphat, Coenzym A, Pyridoxalphosphat, Coenzym B12, Biocytin, Tetrahydrofolat und Liponsäure.

8. Verfahren gemäß einem der Ansprüche 1-7, wobei die prosthetische Gruppe mit dem rekombinanten Polypeptid auf der Oberfläche der Wirtszelle verbunden wird.

9. Verfahren gemäß einem der Ansprüche 1-7, wobei die prosthetische Gruppe mit dem rekombinanten Polypeptid auf einer Membranpräparation stammend aus der Wirtszelle verbunden wird.

10. Verfahren gemäß einem der Ansprüche 1-7, wobei die prosthetische Gruppe mit dem rekombinanten Polypeptid nach Spaltung des rekombinanten Polypeptids von der Wirtszelle oder von einer Membranpräparation davon verbunden wird.

11. Verfahren zur Exposition eines rekombinanten multimeren Polypeptids auf der Oberfläche einer Wirtszelle umfassend die Schritte:
(a) Bereitstellen einer Wirtszelle transformiert mit einer Nukleinsäurefusion operativ verbunden mit einer Expressionskontrollsequenz, wobei die Nukleinsäurefusion umfasst:
(i) einen Anteil kodierend ein Signalpeptid,
(ii) einen Anteil kodierend eine Untereinheit des multimeren Polypeptids, welche exponiert werden soll,
(iii) gegebenenfalls einen Anteil kodierend eine Proteaseerkennungsstelle,
(iv) einen Anteil kodierend einen Transmembranlinker, und
(v) einen Anteil kodierend die Transporterdomäne eines Autotransporters,
(b) Kultivieren der Wirtszelle unter Bedingungen, unter denen die Nukleinsäurefusion exprimiert wird und das Expressionsprodukt umfassend die Untereinheit des multimeren rekombinanten Polypeptids auf der Oberfläche der Wirtszelle exponiert wird, und
(c) Verbinden der exponierten Untereinheit mit wenigstens einer weiteren Untereinheit des multimeren rekombinanten Polypeptids und Bilden eines funktionellen multimeren rekombinanten Polypeptids auf der Oberfläche der Wirtszelle.

12. Verfahren gemäß Anspruch 11, wobei das multimere rekombinante Polypeptid ein Homodimer oder ein Homomultimer ist.

13. Verfahren gemäß Anspruch 11, wobei das multimere rekombinante Polypeptid ein Heterodimer oder ein Heteromultimer ist.

14. Verfahren gemäß einem der Ansprüche 11-13, wobei wenigstens eine Untereinheit des multimeren rekombinanten Proteins eine prosthetische Gruppe enthält.

15. Verfahren gemäß einem der Ansprüche 11-14, wobei ein Homodimer oder ein Homomultimer durch eine Assoziation von mehreren Polypeptiduntereinheiten exponiert auf der Wirtszellmembran gebildet wird.

16. Verfahren gemäß einem der Ansprüche 11-15, wobei ein Heterodimer oder ein Heteromultimer durch eine Assoziation von mehreren verschiedenen Polypeptiduntereinheiten exponiert auf der Wirtszellmembran gebildet wird.

17. Verfahren gemäß einem der Ansprüche 11-14, wobei ein multimeres rekombinantes Polypeptid gebildet wird durch eine Assoziation von wenigstens einer Polypeptiduntereinheit exponiert auf der Wirtszellmembran und wenigstens einer löslichen Polypeptiduntereinheit hinzugefügt zu der Wirtszellmembran.

18. Verfahren gemäß Anspruch 17, wobei die hinzugefügte Untereinheit von der exponierten Untereinheit verschieden ist.

19. Verfahren gemäß einem der vorhergehenden Ansprüche, wobei die Wirtszelle ein Bakterium ist.

20. Verfahren gemäß Anspruch 19, wobei das Bakterium ein gramnegatives Bakterium ist, insbesondere ein Enterobakterium, z. B. *E. coli.*

21. Verfahren gemäß einem der vorhergehenden Ansprüche, wobei die Transporterdomäne des Autotransporters eine β-Fassstruktur bildet.

22. Verfahren gemäß Anspruch 21, wobei die Transporterdomäne des Autotransporters ausgewählt wird aus dem AIDA-I-Protein aus *E. coli,* dem SepA-Protein aus *Shigella flexneri,* dem IcsA-Protein aus *Shigella flexneri,* dem Tsh-Protein aus *E. coli,* dem Ssp-Protein aus *Serratia marcescens,* dem Hsr-Protein aus *Helicobacter mustelae,* dem Prn-Protein aus *Bordetella ssp.,* dem Hap-Protein aus *Haemophilus influenzae,* dem BrkA-Protein aus *Bordetella pertussis,* dem VacA-Protein aus *Helicobacter pylori,* dem Oberflächenprotein SpaP, rOmpB oder SlpT aus *Rickettsia,* der IgA-Protease aus *Neisseria* oder *Haemophilus* und Varianten davon.

23. Verfahren gemäß Anspruch 22, wobei die Transporterdomäne des Autotransporters das AIDA-I-Protein aus *E. coli* oder eine Variante davon ist.

24. Wirtszelle exponierend ein funktionelles rekombinantes Polypeptid auf der Oberfläche, wobei das rekombinante Polypeptid eine prosthetische Gruppe enthält.

25. Wirtszelle gemäß Anspruch 24, wobei das rekombinante Polypeptid durch die Transporterdomäne eines Autotransporters exponiert wird.

26. Wirtszelle gemäß Anspruch 24 oder 25, wobei das Polypeptid ausgewählt wird aus Ferredoxinen, P450-Reductasen, Cytochrom b5, P450-Enzymen, Flavoproteinen und beliebigen Kombinationen davon.

27. Wirtszelle exponierend ein funktionelles rekombinantes Polypeptid auf der Oberfläche, wobei das rekombinante Polypeptid ein multimeres Polypeptid ist, welches wenigstens zwei Polypeptiduntereinheiten enthält.

28. Wirtszelle gemäß Anspruch 27, wobei wenigstens eine Untereinheit des multimeren Polypeptids durch die Transporterdomäne eines Autotransporters exponiert wird.

29. Wirtszelle gemäß einem der Ansprüche 24-28, welche eine bakterielle Wirtszelle, insbesondere eine gramnegative bakterielle Wirtszelle ist.

30. Membranpräparation, welche aus einer Wirtszelle gemäß einem der Ansprüche 24-29 stammt.

31. Verwendung einer Zelle gemäß einem der Ansprüche 24-29 oder einer Membranpräparation gemäß Anspruch 30 für ein chemisches Syntheseverfahren.

32. Verwendung gemäß Anspruch 31 zur Synthese von organischen Substanzen ausgewählt aus Enzymsubstraten, Wirkstoffen, Hormonen, Ausgangsmaterialien und Zwischenprodukten für Syntheseverfahren und chiralen Substanzen.

33. Verwendung einer Zelle gemäß einem der Ansprüche 24-29 oder einer Membranpräparation gemäß Anspruch 30 für ein gerichtetes Evolutionsverfahren.

34. Verwendung einer Zelle gemäß einem der Ansprüche 24-29 oder einer Membranpräparation gemäß Anspruch 30 als ein Testsystem für ein Screeningverfahren, z. B. zur Identifizierung von Modulatoren von P450-Enzymen.

35. Verwendung einer Zelle gemäß einem der Ansprüche 24-29 oder einer Membranpräparation gemäß Anspruch 30 als ein System zur Toxizitätsüberwachung.

36. Verwendung einer Zelle gemäß einem der Ansprüche 24-29 oder einer Membranpräparation gemäß Anspruch 30 als ein System zum Abbau toxischer Substanzen.

## Revendications

1. Procédé pour faire apparaître un polypeptide recombinant contenant un groupement prosthétique à la surface d'une cellule hôte, le procédé comprenant les étapes suivantes :
(a) fournir une cellule hôte transformée avec une fusion d'acide nucléique fonctionnellement liée à une séquence de contrôle de l'expression, ladite fusion d'acide nucléique comprenant
(I) une partie qui code pour un peptide signal,
(II) une partie qui code pour le polypeptide recombinant à faire apparaître,
(III) facultativement une partie qui code pour un site de reconnaissance de protéase,
(IV) une partie qui code pour un lieur transmembranaire, et
(V) une partie qui code pour le domaine transporteur d'un autotransporteur,
(b) cultiver la cellule hôte dans des conditions où la fusion d'acide nucléique est exprimée et où le produit d'expression comprenant le polypeptide recombinant apparaît à la surface de la cellule hôte, et
(c) mettre en contact le polypeptide recombinant avec un groupement prosthétique dans des conditions où le groupement prosthétique se combine au polypeptide recombinant et où est formé un polypeptide recombinant fonctionnel contenant le groupement prosthétique.

2. Procédé selon la revendication 1, dans lequel le groupement prosthétique comprend un composant inorganique.

3. Procédé selon la revendication 2, dans lequel le groupement prosthétique est un groupement qui contient un métal.

4. Procédé selon la revendication 3, dans lequel le métal est choisi parmi le cobalt, le nickel, le manganèse, le cuivre et le fer.

5. Procédé selon la revendication 4, dans lequel le groupement prosthétique est choisi parmi les agrégats [2Fe-2S], les agrégats [4Fe-4S] et la porphyrine métallique, par exemple des groupements hêmes.

6. Procédé selon la revendication 1, dans lequel le groupement prosthétique comprend un composant organique.

7. Procédé selon la revendication 6, dans lequel le groupement prosthétique est choisi parmi les groupements contenant de la flavine, par exemple le FMN ou le FAD, les groupements contenant de la nicotine, par exemple le NAD, le NADH, le NADP ou le NADPH, la biotine, la α2-microglobuline, la thiamine pyrophosphate, le coenzyme A, le pyridoxal phosphate, la coenzyme B 12, le biocytine, le tétrahydrofolate et l'acide lipoïque.

8. Procédé selon l'une des revendications 1 à 7, dans lequel le groupement prosthétique est combiné au polypeptide recombinant à la surface de la cellule hôte.

9. Procédé selon l'une des revendications 1 à 7, dans lequel le groupement prosthétique est combiné au polypeptide recombinant sur une préparation membranaire dérivée de la cellule hôte.

10. Procédé selon l'une des revendications 1 à 7, dans lequel le groupement prosthétique est combiné au polypeptide recombinant après que le polypeptide recombinant a été clivé de la cellule hôte ou d'une préparation membranaire de celle-ci.

11. Procédé pour faire apparaître un polypeptide multimérique recombinant à la surface d'une cellule hôte, le procédé comprenant les étapes suivantes :
(a) fournir une cellule hôte transformée avec une fusion d'acide nucléique fonctionnellement liée à une séquence de contrôle de l'expression, ladite fusion d'acide nucléique comprenant
(I) une partie qui code pour un peptide signal,
(II) une partie qui code pour une sous-unité du polypeptide multimérique à faire apparaître,
(III) facultativement une partie qui code pour un site de reconnaissance de protéase,
(IV) une partie qui code pour un lieur transmembranaire, et
(V) une partie qui code pour le domaine transporteur d'un autotransporteur,
(b) cultiver la cellule hôte dans des conditions où la fusion d'acide nucléique est exprimée et où le produit d'expression comprenant la sous-unité du polypeptide multimérique recombinant apparaît à la surface de la cellule hôte, et
(c) combiner la sous-unité qui est apparue à au moins une autre sous-unité du polypeptide multimérique recombinant et former un polypeptide multimérique recombinant fonctionnel à la surface de la cellule hôte.

12. Procédé selon la revendication 11, dans lequel le polypeptide multimérique recombinant est un homodimère ou un homomultimère.

13. Procédé selon la revendication 11, dans lequel le polypeptide multimérique recombinant est un hétérodimère ou un hétéromultimère.

14. Procédé selon l'une des revendications 11 à 13, dans lequel au moins une sous-unité de la protéine multimérique recombinante contient un groupement prosthétique.

15. Procédé selon l'une des revendications 11 à 14, dans lequel un homodimère ou un homomultimère est formé en associant plusieurs sous-unités de polypeptide apparues à la membrane de la cellule hôte.

16. Procédé selon l'une des revendications 11 à 15, dans lequel un hétérodimère ou un hétéromultimère est formé en associant plusieurs sous-unités de polypeptide différentes apparues sur la membrane de la cellule hôte.

17. Procédé selon l'une des revendications 11 à 14, dans lequel un polypeptide multimérique recombinant est formé en associant au moins une sous-unité de polypeptide apparue sur la membrane de la cellule hôte et au moins une sous-unité de polypeptide soluble ajoutée à la membrane de la cellule hôte.

18. Procédé selon la revendication 17, dans lequel la sous-unité ajoutée est différente de la sous-unité apparue.

19. Procédé selon l'une des revendications précédentes, dans lequel la cellule hôte est une bactérie.

20. Procédé selon la revendication 19, dans lequel la bactérie est une bactérie Gram-négative, en particulier une entérobactérie, par exemple *E. coli.*

21. Procédé selon l'une des revendications précédentes, dans lequel le domaine transporteur de l'autotransporteur forme une structure de tonneau β.

22. Procédé selon la revendication 21, dans lequel le domaine transporteur de l'autotransporteur est choisi parmi la protéine AIDA-I d'*E. coli,* la protéine Sep A de *Shigella flexneri,* la protéine IcsA de *Shigella flexneri,* la protéine Tsh d'*E. coli,* la protéine Ssp de *Serraria marcescens,* la protéine Hsr de *Helicobacter mustelae,* la protéine Pm de *Bordetella ssp.,* la protéine Hap de *Haemophilus influenzae,* la protéine Brk A de *Bordetalla pertussis,* la protéine Vac A de *Helicobacter pylori,* la protéine de surface SpaP, rOmpB ou SIpT de *Rickettsia,* la protéase IgA de *Neisseria* ou de *Haemophilus* ainsi que leurs variantes.

23. Procédé selon la revendication 22, dans lequel le domaine transporteur de l'autotransporteur est la protéine AIDA-I d'*E*. *coli* ou une de ses variantes.

24. Cellule hôte avec un polypeptide recombinant fonctionnel apparaissant à sa surface, le polypeptide recombinant contenant un groupement prosthétique.

25. Cellule hôte selon la revendication 24, dans laquelle le domaine transporteur d'un autotransporteur fait apparaître le polypeptide recombinant.

26. Cellule hôte selon la revendication 24 ou 25, dans laquelle le polypeptide est choisi parmi les ferrodoxines, les réductases P 450, le cytochrome b5, les enzymes P 450, les flavoprotéines et toutes leurs combinaisons.

27. Cellule hôte avec un polypeptide recombinant fonctionnel apparaissant à sa surface, le polypeptide recombinant étant un polypeptide multimérique qui contient au moins deux sous-unités de polypeptide.

28. Cellule hôte selon la revendication 27, dans laquelle le domaine transporteur d'un autotransporteur fait apparaître au moins une sous-unité du polypeptide multimérique.

29. Cellule hôte selon l'une des revendications 24 à 28, qui est une cellule hôte bactérienne, en particulier une cellule hôte bactérienne Gram-négative.

30. Préparation de membrane dérivée d'une cellule hôte selon l'une des revendications 24 à 29.

31. Utilisation d'une cellule selon l'une des revendications 24 à 29 ou d'une préparation de membrane selon la revendication 30 pour un procédé de synthèse chimique.

32. Utilisation selon la revendication 31 pour synthétiser des substances organiques choisies parmi les substrats enzymatiques, les médicaments, les hormones, les produits de départ et les produits intermédiaires pour des procédés de synthèse et les substances chirales.

33. Utilisation d'une cellule selon l'une des revendications 24 à 29 ou d'une préparation de membrane selon la revendication 30 pour un procédé d'évolution dirigée.

34. Utilisation d'une cellule selon l'une des revendications 24 à 29 ou d'une préparation de membrane selon la revendication 30 comme système de dosage pour un procédé de crible, par exemple pour identifier des modulateurs des enzymes P 450.

35. Utilisation d'une cellule selon l'une des revendications 24 à 29 ou d'une préparation de membrane selon la revendication 30 comme système pour contrôler la toxicité.

36. Utilisation d'une cellule selon l'une des revendications 24 à 29 ou d'une préparation de membrane selon la revendication 30 comme système pour dégrader des substances toxiques.


### SEQUENCE LISTING

<110> Max-Planck-Gesellschaft zur Förderung der Wissensc
<120> Functional surface display of polypeptides
<130> 24171PWO_DR
<140>
   <141>
<150> 01 105 129.9
   <151> 2001-03-02
<160> 9
<170> PatentIn Ver. 2.1
<210> 1
   <211> 396
   <212> DNA
   <213> Bovine
<220>
   <221> CDS
   <222> (1)..(396)
   <223> bovine adrenodoxine, devoid of the mitochondrial target sequence as used in this study
<400> 1
   ctc gag ggc agc tca gaa gat aaa ata aca gtc cac ttt ata aac cgt   48
   Leu Glu Gly Ser Ser Glu Asp Lys Ile Thr Val His Phe Ile Asn Arg
   1               5                   10                  15
   gat ggt gaa aca tta aca acc aaa gga aaa att ggt gac tct ctg cta   96
   Asp Gly Glu Thr Leu Thr Thr Lys Gly Lys Ile Gly Asp Ser Leu Leu
               20                  25                  30
   gat gtt gtg gtt caa aat aat cta gat att gat ggt ttt ggt gca tgt   144
   Asp Val Val Val Gln Asn Asn Leu Asp Ile Asp Gly Phe Gly Ala Cys
           35                  40                  45
   gag gga acc ttg gct tgt tct acc tgt cac ctc atc ttt gaa cag cac   192
   Glu Gly Thr Leu Ala Cys Ser Thr Cys His Leu Ile Phe Glu Gln His
       50                  55                  60
   ata ttt gag aaa ttg gaa gca atc act gat gag gag aat gac atg ctt   240
   Ile Phe Glu Lys Leu Glu Ala Ile Thr Asp Glu Glu Asn Asp Met Leu
   65                  70                  75                  80
   gat ctg gca tat gga cta aca gat aga tcg cgg ttg ggc tgc cag atc   288
   Asp Leu Ala Tyr Gly Leu Thr Asp Arg Ser Arg Leu Gly Cys Gln Ile
                   85                  90                  95
   tgt ttg aca aag gct ntg gac aat atg act gtt cga gta cct gat gcc   336
   Cys Leu Thr Lys Ala Xaa Asp Asn Met Thr Val Arg Val Pro Asp Ala
               100                 105                 110
   gta tct gat gcc aga gag tcc att gat atg ggc atg aac tcc tca aag   384
   Val Ser Asp Ala Arg Glu Ser Ile Asp Met Gly Met Asn Ser Ser Lys
           115                 120                 125
   ata gaa ggt acc                                                   396
   Ile Glu Gly Thr
       130
<210> 2
   <211> 132
   <212> PRT
   <213> Bovine
<400> 2
   Leu Glu Gly Ser Ser Glu Asp Lys Ile Thr Val His Phe Ile Asn Arg
   1               5                   10                  15
   Asp Gly Glu Thr Leu Thr Thr Lys Gly Lys Ile Gly Asp Ser Leu Leu
               20                  25                  30
   Asp Val Val Val Gln Asn Asn Leu Asp Ile Asp Gly Phe Gly Ala Cys
           35                  40                  45
   Glu Gly Thr Leu Ala Cys Ser Thr Cys His Leu Ile Phe Glu Gln His
       50                  55                  60
   Ile Phe Glu Lys Leu Glu Ala Ile Thr Asp Glu Glu Asn Asp Met Leu
   65                  70                  75                  80
   Asp Leu Ala Tyr Gly Leu Thr Asp Arg Ser Arg Leu Gly Cys Gln Ile
                   85                  90                  95
   Cys Leu Thr Lys Ala Xaa Asp Asn Met Thr Val Arg Val Pro Asp Ala
               100                 105                 110
   Val Ser Asp Ala Arg Glu Ser Ile Asp Met Gly Met Asn Ser Ser Lys
           115                 120                 125
   Ile Glu Gly Thr
       130
<210> 3
   <211> 54
   <212> DNA
   <213> Artificial Sequence
<220>
   <223> Description of Artificial Sequence: ctxB signal peptide
<220>
   <221> CDS
   <222> (1)..(54)
   <223> translation of the ctxB signal peptide
<400> 3
   tat gca cat gga aca cct caa aat att act gat ttg ctc gag ggc agc    48
   Tyr Ala His Gly Thr Pro Gln Asn Ile Thr Asp Leu Leu Glu Gly Ser
     1               5                  10                  15
   tca gaa                                                            54
   Ser Glu
<210> 4
   <211> 18
   <212> PRT
   <213> Artificial Sequence
   <223> Description of Artificial Sequence: ctxB signal peptide
<400> 4
   Tyr Ala His Gly Thr Pro Gln Asn Ile Thr Asp Leu Leu Glu Gly Ser
     1               5                  10                  15
   Ser Glu
<210> 5
   <211> 51
   <212> DNA
   <213> Artificial Sequence
<220>
   <223> Description of Artificial Sequence: linker peptide
<220>
   <221> CDS
   <222> (1)..(51)
   <223> translation of the linker peptide
<400> 5
   gat atg ggc atg aac tcc tca aag ata gaa ggt acc ctt aat cct aca    48
   Asp Met Gly Met Asn Ser Ser Lys Ile Glu Gly Thr Leu Asn Pro Thr
     1               5                  10                  15
   aaa                                                                51
   Lys
<210> 6
   <211> 17
   <212> PRT
   <213> Artificial Sequence
   <223> Description of Artificial Sequence: linker peptide
<400> 6
   Asp Met Gly Met Asn Ser Ser Lys Ile Glu Gly Thr Leu Asn Pro Thr
   1 5 10 15
   Lys
<210> 7
   <211> 623
   <212> PRT
   <213> Coriolusolor
<400> 7
   Met Ser Thr Ser Ser Ser Asp Pro Phe Phe Asn Phe Thr Lys Ser Ser
   1 5 10 15
   Phe Arg Ser Ala Ala Ala Gln Lys Ala Ser Ala Thr Ser Leu Pro Pro
   20 25 30
   Leu Pro Gly Pro Asp Lys Lys Val Pro Gly Met Asp Ile Lys Tyr Asp
   35 40 45
   Val Val Ile Val Gly Ser Gly Pro Ile Gly Cys Thr Tyr Ala Arg Glu
        50                  55                  60
   Leu Val Glu Ala Gly Tyr Lys Val Ala Met Phe Asp Ile Gly Glu Ile
   65                  70                  75                  80
   Asp Ser Gly Leu Lys Ile Gly Ala His Lys Lys Asn Thr Val Glu Tyr
                   85                  90                  95
   Gln Lys Asn Ile Asp Lys Phe Val Asn Val Ile Gln Gly Gln Leu Met
               100                 105                 110
   Ser Val Ser Val Pro Val Asn Thr Leu Val Ile Asp Thr Leu Ser Pro
           115                 120                 125
   Thr Ser Trp Gln Ala Ser Ser Phe Phe Val Arg Asn Gly Ser Asn Pro
       130                 135                 140
   Glu Gln Asp Pro Leu Arg Asn Leu Ser Gly Gln Ala Val Thr Arg Val
   145                 150                 155                 160
   Val Gly Gly Met Ser Thr His Trp Thr Cys Ala Thr Pro Arg Phe Asp
                   165                 170                 175
   Arg Glu Gln Arg Pro Leu Leu Val Lys Asp Asp Gln Asp Ala Asp Asp
               180                 185                 190
   Ala Glu Trp Asp Arg Leu Tyr Thr Lys Ala Glu Ser Tyr Phe Lys Thr
           195                 200                 205
   Gly Thr Asp Gln Phe Lys Glu Ser Ile Arg His Asn Leu Val Leu Asn
       210                 215                 220
   Lys Leu Ala Glu Glu Tyr Lys Gly Gln Arg Asp Phe Gln Gln Ile Pro
   225                 230                 235                 240
   Leu Ala Ala Thr Arg Arg Ser Pro Thr Phe Val Glu Trp Ser Ser Ala
                   245                 250                 255
   Asn Thr Val Phe Asp Leu Gln Asn Arg Pro Asn Thr Asp Ala Pro Asn
               260                 265                 270
   Glu Arg Phe Asn Leu Phe Pro Ala Val Ala Cys Glu Arg Val Val Arg
           275                 280                 285
   Asn Thr Ser Asn Ser Glu Ile Glu Ser Leu His Ile His Asp Leu Ile
       290                 295                 300
   Ser Gly Asp Arg Phe Glu Ile Lys Ala Asp Val Phe Val Leu Thr Ala
   305                 310                 315                 320
   Gly Ala Val His Asn Ala Gln Leu Leu Val Asn Ser Gly Phe Gly Gln
                   325                 330                 335
   Leu Gly Arg Pro Asp Pro Ala Asn Pro Pro Gln Leu Leu Pro Ser Leu
               340                 345                 350
   Gly Ser Tyr Ile Thr Glu Gln Ser Leu Val Phe Cys Gln Thr Val Met
           355                 360                 365
   Ser Thr Glu Leu Ile Asp Ser Val Lys Ser Asp Met Ile Ile Arg Gly
       370                 375                 380
   Asn Pro Gly Asp Leu Gly Tyr Ser Val Thr Tyr Thr Pro Gly Ala Glu
   385                 390                 395                 400
   Thr Asn Lys His Pro Asp Trp Trp Asn Glu Lys Val Lys Asn His Met
                   405                 410                 415
   Met Gln His Gln Glu Asp Pro Leu Pro Ile Pro Phe Glu Asp Pro Glu
               420                 425                 430
   Pro Gln Val Thr Thr Leu Phe Gln Pro Ser His Pro Trp His Thr Gln
           435                 440                 445
   Ile His Arg Asp Ala Phe Ser Tyr Gly Ala Val Gln Gln Ser Ile Asp
       450                 455                 460
   Ser Arg Leu Ile Val Asp Trp Arg Phe Phe Gly Arg Thr Glu Pro Lys
   465                 470                 475                 480
   Glu Glu Asn Lys Leu Trp Phe Ser Asp Lys Ile Thr Asp Thr Tyr Asn
                   485                 490                 495
   Met Pro Gln Pro Thr Phe Asp Phe Arg Phe Pro Ala Gly Arg Thr Ser
               500                 505                 510
   Lys Glu Ala Glu Asp Met Met Thr Asp Met Cys Val Met Ser Ala Lys
           515                 520                 525
   Ile Gly Gly Phe Leu Pro Gly Ser Leu Pro Gln Phe Met Glu Pro Gly
       530                 535                 540
   Leu Val Leu His Leu Gly Gly Thr His Arg Met Gly Phe Asp Glu Gln
   545                 550                 555                 560
   Glu Asp Lys Cys Cys Val Asn Thr Asp Ser Arg Val Phe Gly Phe Lys
   565 570 575
   Asn Leu Phe Leu Gly Gly Cys Gly Asn Ile Pro Thr Ala Tyr Gly Ala
   580 585 590
   Asn Pro Thr Leu Thr Ala Met Ser Leu Ala Ile Lys Ser Cys Glu Tyr
   595 600 605
   Ile Lys Asn Asn Phe Thr Pro Ser Pro Phe Thr Asp Gln Ala Glu
   610 615 620
<210> 8
   <211> 36
   <212> DNA
   <213> Artificial Sequence
<220>
   <223> Description of Artificial Sequence: oligonucleotide primer JJ3
<400> 8
   ccgctcgagg gcagctcaga agataaaata acagtc 36
<210> 9
   <211> 30
   <212> DNA
   <213> Artificial Sequence
<220>
   <223> Description of Artificial Sequence: oligonucleotide primer JJ4
<400> 9
   ggggtacctt ctatctttga ggagttcatg 30